# EUROPEAN PATENT SPECIFICATION

(11) **EP 1 077 981 B1**
(45) Date of publication and mention of the grant of the patent: **07.08.2002**
(21) Application number: 99919695.9
(22) Date of filing: 12.05.1999
(51) Int. Cl.: C07D 501/00, C07D 501/59, A61K 31/545

(54) **NOVEL CEPHALOSPORIN COMPOUNDS, PROCESSES FOR PREPARATION THEREOF AND ANTIMICROBIAL COMPOSITIONS CONTAINING THE SAME**
CEPHALOSPORIN-DERIVATE, VERFAHREN ZU IHRER HERSTELLUNG UND DIESE ENTHALTENDE ANTIMIKROBIELLE MITTEL
NOUVEAUX COMPOSES DE CEPHALOSPORINE, PROCEDES DE PREPARATION DESDITS COMPOSES ET COMPOSITIONS ANTIMICROBIENNES CONTENANT CES COMPOSES

(30) Priority: 13.05.1998 KR 9817260; 31.08.1998 KR 9835650; 31.08.1998 KR 9835651; 04.02.1999 KR 9903757
(43) Date of publication of application: 28.02.2001
(73) Proprietor: LG CHEMICAL LIMITED, Seoul 150-010 (KR)
(72) Inventor: LEE, Chang Seok, Yuseong-ku, Daejeon 305-340 (KR); OH, Seong Ho, Yuseong-ku, Daejeon 305-345 (KR); MOON, Kwang Yul, Seo-ku, Daejeon 302-150 (KR); RYU, Eun Jung, Taeyoung Moogoonghwa, Seo-ku, Daejeon 302-280 (KR); KIM, Yong Zu, Yuseong-ku, Daejeon 305-345 (KR); KIM, Mu Yong, Yuseong-ku, Daejeon 305-340 (KR); PAEK, Kyoung Sook, Yuseong-ku, Daejeon 305-340 (KR); LEE, Sun Hwa, Chungkoonarae, Yuseong-ku, Daejeon 305-390 (KR)
(74) Representative: Kaiser, Jürgen, Dr.rer.nat.Dipl.-Chem.
(86) International application number: KR9900236
(87) International publication number: WO9958535

(56) References cited:
- EP-A2- 0 210 078
- EP-A2- 0 329 457
- WO-A-95/07283
- US-A- 4 482 565

## Description

The present invention relates to novel cephalosporin compounds which are useful as antibiotics. More specifically, the present invention relates to novel cephalosporin compounds of the following formula (I): 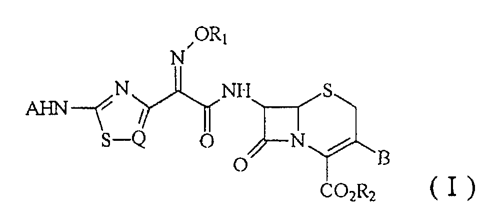 , wherein
A represents hydrogen or amino protecting group,
R₁ represents hydrogen, C₁₋₆alkyl which may be mono- to tri-substituted by C₁₋₆alkoxy, cyano, halogen, C₃₋₆cycloalkyl or C₅₋₆heteroaryl having 1 or 2 nitrogen or oxygen atom(s), C₃₋₄alkenyl, C₃₋₄alkynyl which may be substituted by amino, or C₃₋₆cycloalkyl,
R₂ represents hydrogen or carboxy protecting group,
Q represents CH, CX or N, wherein X represents halogen,
B represents a radical selected from the following group: 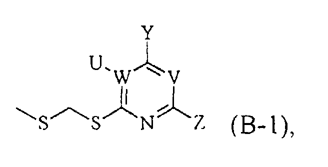 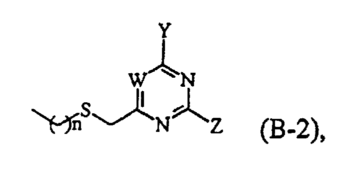 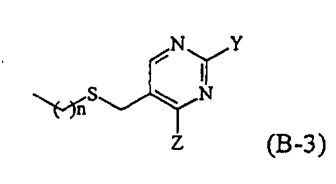 or 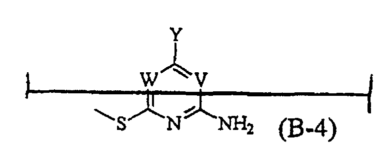 , wherein
   U represents hydrogen or optionally substituted amino,
   V and W independently of one another represent C or N, with the proviso that V and W are different,
   Y and Z independently of one another represent hydrogen, optionally substituted amino, hydroxy or C₁₋₄alkyl, and
   n is 0 or 1,
pharmaceutically acceptable nontoxic salts, physiologically hydrolyzable esters, hydrates, solvates and isomers thereof, which exhibit potent antimicrobial activity, and have broad antibacterial spectrum and remarkably improved pharmacokinetic characteristics, and are also effective for oral administration.

The present invention also provides processes for preparation thereof and antimicrobial compositions containing the same as active ingredients.

### BACKGROUND ART

Cephalosporin antibiotics have been widely used for treatment of infectious diseases caused by pathogenic bacteria in human beings and animals. In particular, they are useful for treatment of diseases caused by bacteria having a resistance to other antibiotics such as penicillin and for treatment of the diseases in penicillin-hypersensitive patients. For treatment of such infectious diseases, in almost cases, it is preferable to use antibiotics having antimicrobial activity not only against Gram positive bacteria but also against Gram negative bacteria.

It is well known that antimicrobial activity of cephalosporin antibiotics is largely dependent on the substituents present in 3- or 7-position of cephem ring. Accordingly, in order to develop antibiotics having potent antimicrobial activity against Gram positive bacteria and against Gram negative bacteria, numerous cephalosporin antibiotics have been developed hitherto which have various substituents in 3- or 7-position of cephem ring.

For example, GB patent No. 1,399,086 disclosed cephalosporin derivatives of the following formula (II):

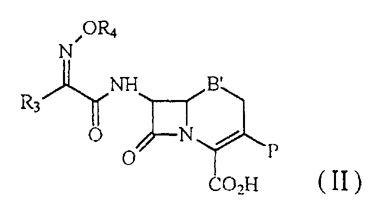

In the above formula (II),
R₃ represents hydrogen or organic group,
R₄ represents etherified monovalent organic group which is attached to oxygen via carbon,
B' represents -S- or >S→O, and
P represents organic group.

Since these compounds were developed, numerous studies have been performed to develop antibiotics exhibiting improved antimicrobial activity against Gram negative bacteria. For example, GB patent No. 1,522,140 disclosed cephalosporin antibiotics of the following formula (III) which are syn- isomers or mixtures of syn- and anti- isomers containing 90% or more of syn- isomers:

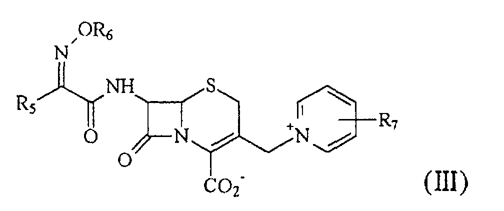

In the above formula (III),
R₅ represents furyl or thienyl,
R₆ represents C₁₋₄alkyl, C₃₋₄cycloalkyl, furylmethyl or thienylmethyl, and
R₇ represents hydrogen, carbamoyl, carboxymethyl, sulfonyl or methyl.

Thereafter, numerous studies were performed to develop antibiotics having broad antimicrobial spectrum which exhibit improved antimicrobial activity not only against Gram negative bacteria but also against Gram positive bacteria. As a result, many cephalosporin antibiotics having similar structures to the above formula (III) were developed. These compounds were developed by means of various modifications, for example, incorporation of acylamido group into C-7 position of cephem ring, incorporation of a specific group into C-3 position of cephem ring, and the like.

For example, Belgian patent No. 852,427 disclosed cephalosporin compounds in which R₃ is substituted by other various organic groups, for example, 2-aminothiazol-4-yl, in the above formula (II), oxygen atom of oxyimino group is attached to aliphatic hydrocarbon group and the aliphatic hydrocarbon group itself may be substituted by carboxy group. In the above compounds, the substituent present in C-3 position is acyloxymethyl, hydroxymethyl, formyl or optionally substituted heterocyclic thiomethyl group.

Recently, as antibiotics not only exhibiting potent activity against a wide range of pathogenic bacteria but also being sustained at a high concentration in blood for a long time to be orally administrable, compounds were developed in which thioalkylthio, thioalkyl or heteroarylthio group was incorporated into C-3 position.

Specifically, the United States patent No. 5,214,037 disclosed cephalosporin derivatives of the following formula (IV-a) which exhibit antimicrobial activity against a wide range of pathogenic bacteria by incorporation of thioalkylthio chain into C-3 position:

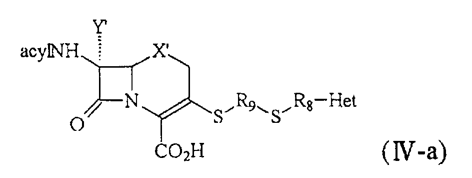

In the above formula (IV-a),
acyl represents C₁-C₁₂ acyl,
Het represents monocyclic heteroaromatic group having one or more hetero atoms(particularly, pyridyl, 1,2,3- or 1,2,4-triazolyl, 1,2,3- or 1,3,4- thiadiazolyl or tetrazolyl),
R₈ represents single bond or C₁-C₄alkylene,
R₉ represents C₁-C₄alkylene,
X' represents sulfur atom or sulfoxide, and
Y' represents hydrogen or methoxy group.

In the above-mentioned patent, various kinds of heteroaromatic rings are incorporated into C-3 thioalkyl chain but they are different with hetero rings of the present compounds. That is, the compounds of the present invention are characterized in that they have optionally substituted pyrimidinyl group in C-3 position but such compounds have not been described in the above patent.

In addition, International publication No. WO 95-07283 disclosed cephalosporin derivatives of the following formula (IV-b) which exhibit improved antimicrobial activity against a wide range of pathogenic bacteria by incorporation of thioalkyl chain into C-3 position:

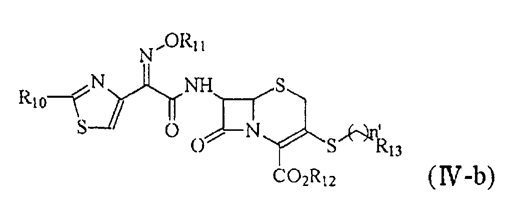

In the above formula (IV-b),
R₁₀ represents amino or protected amino,
R₁₁ represents hydrogen, lower alkyl or hydroxy protecting group,
R₁₂ represents hydrogen or carboxy protecting group,
R₁₃ represents 3-pyridyl, 4-pyridyl or optionally substituted heteromonocyclic ring having 2 nitrogen atoms, 1 oxygen or sulfur atom,
n' is 0, 1 or 2, and
provided that R₁₁ represents lower alkyl, n' is 1 or 2.

Compounds disclosed in the above patent have various kinds of heteroaromatic rings in thioalkyl chain of C-3 position but the heteroaromatic rings are different with hetero rings of the compounds according to the present invention. That is, the compounds of the present invention are characterized by having optionally substituted pyrimidinyl group in C-3 position but such compounds have not been described in the above patent.

Also, the United States patent No. 5,362,722 disclosed cephalosporin derivatives of the following formula (IV-c) which have improved antimicrobial activity against a wide range of pathogenic bacteria by incorporation of substituted thiazolylthio group into C-3 position:

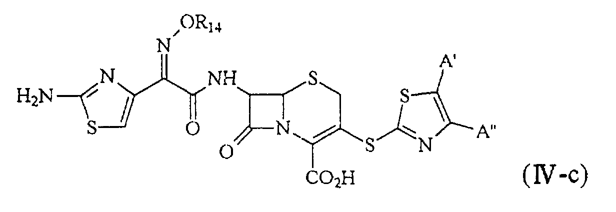

In the above formula (IV-c),
R₁₄ represents hydrogen, C₁-C₆alkyl, C₂-C₆alkenyl, C₂-C₆alkynyl, C₃-C₆cycloalkyl or C₁-C₆haloalkyl, and
A' and A" independently of one another represent hydrogen, C₁-C₆alkyl, nitro, amino, or 5- or 6-membered cyclic compound having 1 to 3 hetero atom(s).

The compounds of the above formula (IV-c) have thiazole group in C-3 position but the thiazole group is different with amino-substituted pyrimidine ring of the compounds according to the present invention. However, these compounds cannot exhibit sufficiently antimicrobial activity against a wide range of pathogenic bacteria including Gram positive bacteria, have undesirable pharmacokinetic characteristics and particularly, cannot be applied for oral administration.

### DISCLOSURE OF THE INVENTION

The present inventors have performed extensive studies to develop cephalosporin compounds which exhibit potent antimicrobial activity against a wide range of pathogenic bacteria including Gram positive bacteria, have improved pharmacokinetic characteristics and particularly, are also effective for oral administration. As a result, they discovered that cephalosporin compounds having optionally substituted pyrimidinyl group in C-3 position can attain such objects and finally, completed the present invention.

The present invention provides novel cephalosporin compounds of the following formula (I):

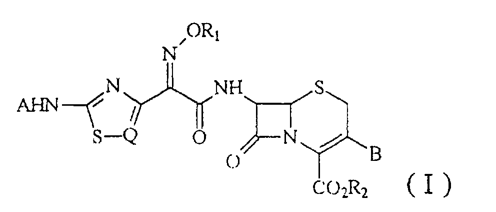

In the above formula ( I ),
A represents hydrogen or amino protecting group,
R₁ represents hydrogen, C₁₋₆alkyl which may be mono- to tri-substituted by C₁₋₆alkoxy, cyano, halogen, C₃₋₆cycloalkyl or C₅₋₆heteroaryl having 1 or 2 nitrogen or oxygen atom(s), C₃₋₄alkenyl. C₃₋₄alkynyl which may be substituted by amino, or C₃₋₆cycloalkyl,
R₂ represents hydrogen or carboxy protecting group,
Q represents CH, CX or N, wherein X represents halogen,
B represents a radical selected from the following group: 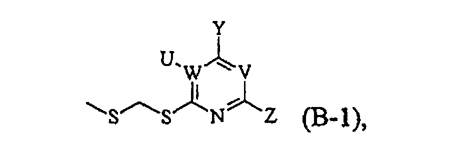 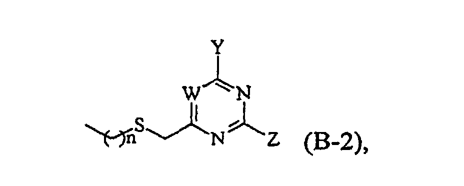 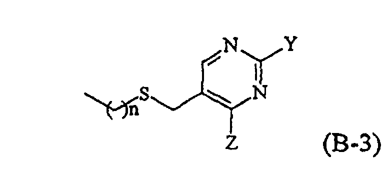 or 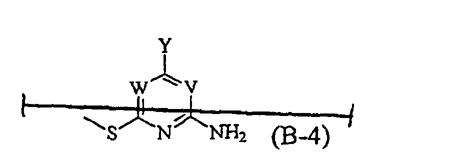 , wherein
   U represents hydrogen or optionally substituted amino,
   V and W independently of one another represent C or N, with the proviso that V and W are different,
   Y and Z independently of one another represent hydrogen, optionally substituted amino, hydroxy or C₁₋₄alkyl, and
   n is 0 or 1.
, pharmaceutically acceptable nontoxic salts, physiologically hydrolyzable esters, hydrates, solvates and isomers thereof.

The present invention also provides processes for preparation of the compounds of the formula (I) and antimicrobial compositions containing the same as active ingredients.

### BEST MODE FOR CARRYING OUT THE INVENTION

Representative examples of the compounds of the formula (I) according to the present invention are as follows:
7-[(Z)-2-(2-amino-thiazol-4-yl)-2-methoxyimino-acetylamino]-8-oxo-3 -(pyrimidin-2-ylsulfanylmethylsulfanyl)-5-thia-1-aza-bicyclo[4,2,0]oct-2-ene-2 -carboxylic acid(1);
7-[(Z)-2-(2-amino-thiazol-4-yl)-2-methoxyimino-acetylamino]-8-oxo-3 -(4-amino-pyrimidin-2-ylsulfanylmethylsulfanyl)-5-thia-1-aza-bicyclo[4,2,0]oc t-2-ene-2-carboxylic acid(2);
7-[(Z)-2-(2-amino-thiazol-4-yl)-2-methoxyimino-acetylamino]-3-(4,6-d iamino-pyrimidin-2-ylsulfanylmethylsulfanyl)-8-oxo-5-thia-1-aza-bicyclo[4,2,0 ]oct-2-ene-2-carboxylic acid(3);
7-[(Z)-2-(2-amino-thiazol-4-yl)-2-methoxyimino-acetylamino]-3-(2,6-d iamino-pyrimidin-4-ylsulfanylmethylsulfanyl)-8-oxo-5-thia-1-aza-bicyclo[4,2,0 ]oct-2-ene-2-carboxylic acid(4);
7-[(Z)-2-(2-amino-thiazol-4-yl)-2-hydroxyimino-acetylamino]-3-(4,6-di amino-pyrimidin-2-ylsulfanylmethylsulfanyl)-8-oxo-5-this-1-aza-bicyclo[4,2,0] oct-2-ene-2-carboxylic acid(5);
7-[(Z)-2-(2-amino-thiazol-4-yl)-2-hydroxyimino-acetylamino]-3-(2,6-di amino-pyrimidin-4-ylsulfanylmethylsulfanyl)-8-oxo-5-thia-1-aza-bicyclo[4,2,0] oct-2-ene-2-carboxylic acid(6);
7-[(Z)-2-(2-amino-5-chloro-thiazol-4-yl)-2-hydroxyimino-acetylamino] -3-(2,6-diamino-pyrimidin-4-ylsulfanylmethylsulfanyl)-8-oxo-5-thia-1-aza-bicy clo[4,2,0]oct-2-ene-2-carboxylic acid(7);
7-[(Z)-2-(2-amino-5-chloro-thiazol-4-yl)-2-methoxyimino-acetylamino] -3-(2,6-diamino-pyrimidin-4-ylsulfanylmethylsulfanyl)-8-oxo-5-thia-1-aza-bicy clo[4,2,0]oct-2-ene-2-carboxylic acid(8);
7-[(Z)-2-(2-amino-thiazol-5-chloro-4-yl)-2-hydroxyimino-acetylamino] -3-(2-amino-6-methyl-pyrimidin-4-ylsulfanylmethylsulfanyl)-8-oxo-5-thia-1-az a-bicyclo[4,2,0]oct-2-ene-2-carboxylic acid(9);
7-[(Z)-2-(2-amino-thiazol-5-chloro-4-yl)-2-hydroxyimino-acetylamino] -3-(4-amino-pyrimidin-2-ylsulfanylmethylsulfanyl)-8-oxo-5-thia-1-aza-bicyclo[ 4,2,0]oct-2-ene-2-carboxylic acid(10);
7-[(Z)-2-(2-amino-thiazol-5-chloro-4-yl)-2-hydroxyimino-acetylamino] -3-(2-amino-6-hydroxy-pyrimidin-4-ylsulfanyhnethylsulfanyl)-8-oxo-5-thia-1-aza-bicyclo[4,2,0]oct-2-ene-2-carboxylic acid(11);
7-[(Z)-2-(2-amino-thiazol-5-chloro-4-yl)-2-hydroxyimino-acetylamino] -3-(4,6-diamino-pyrimidin-2-ylsulfanylmethylsulfanyl)-8-oxo-5-thia-1-aza-bicy clo[4,2,0]oct-2-ene-2-carboxylic acid(12);
7-[(Z)-2-(2-amino-thiazol-5-chloro-4-yl)-2-hydroxyimino-acetylamino] -3-(4-amino-6-hydroxy-pyrimidin-2-ylsulfanylmethylsulfanyl)-8-oxo-5-thia-1-aza-bicyclo[4,2,0]oct-2-ene-2-carboxylic acid(13);
7-[(Z)-2-(2-amino-thiazol-5-chloro-4-yl)-2-hydroxyimino-acetylamino] -3-(4,5-diamino-2-hydroxy-pyrimidin-6-ylsulfanylmethylsulfanyl)-8-oxo-5-thia -1-aza-bicyclo[4,2,0]oct-2-ene-2-carboxylic acid(14);
7-[(Z)-2-(2-amino-thiazol-5-chloro-4-yl)-2-hydroxyimino-acetylamino] -3-(2,5-diamino-6-hydroxy-pyrimidin-4-ylsulfanylmethylsulfanyl)-8-oxo-5-thia -1-aza-bicyclo[4,2,0]oct-2-ene-2-carboxylic acid(15);
7-{[2-(2-amino-5-chloro-1,3-thiazol-4-yl)-2-(methoxyimino)acetyl]ami no}-3-({[(2,6-diamino-4-pyrimidinyl)sulfanyl]methyl}sulfanyl)-8-oxo-5-thia-1 -aza-bicyclo[4,2,0]oct-2-ene-2-carboxylic acid (E)-2-(ethoxycarbonyl)-2-pent enyl ester( 16);
7-{[2-(2-amino-5-chloro-1,3-thiazol-4-yl)-2-(methoxyimino)acetyl]ami no}-3-({[(2,6-diamino-4-pyrimidinyl)sulfanyl]methyl}sulfanyl)-8-oxo-5-thia-1 -aza-bicyclo[4,2,0]oct-2-ene-2-carboxylic acid [(2,2-dimethylpropanoyl)oxy] methyl ester(17);
7-{[2-(2-{[(2S}-2-amino-2-phenylacetyl]amino}-5-chloro-1,3-thiazol-4-yl)-2-(methoxyimino)acetyl]amino}-3-({[(2,6-diarnino-4-pyrimidinyl)sulfanyl] methyl}sulfanyl)-8-oxo-5-thia-1-aza-bicyclo[4,2,0]oct-2-ene-2-carboxylic acid (18);
7-[(Z)-2-(2-amino-1,3-thiazol-4-yl)-2-methoxyimino-acetylamino]-3-(2 ,4-diaminopyrimidin-5-ylmethylsulfanyl)-8-oxo-5-thia-1-aza-bicyclo[4,2,0]oct-2-ene-2-carboxylic acid(19);
7-[(Z)-2-(2-amino-1,3-thiazol-4-yl)-2-methoxyimino-acetylamino]-3-(2 ,4-diammopyrimidin-6-ylmethylsulfanyl)-8-oxo-5-thia-1-aza-bicyclo[4,2,0]oct-2-ene-2-carboxylic acid(20);
7-[(Z)-2-(2-amino-5-chloro-1,3-thiazol-4-yl)-2-methoxyimino-acetylam ino]-3-(2,4-diaminopyrimidin-5-ylmethylsulfanyl)-8-oxo-5-thia-1-aza-bicyclo[ 4,2,0]oct-2-ene-2-carboxylic acid(21);
7-[(Z)-2-(2-amino-5-chloro-1,3-thiazol-4-yl)-2-hydroxyimino-acetylami no]-3-(2,4-diaminopyrimidin-6-ylmethylsulfanyl)-8-oxo-5-thia-1-aza-bicyclo[4 ,2,0]oct-2-ene-2-carboxylic acid(22);
7-[(Z)-2-(2-amino-1,3-thiazol-4-yl)-2-methoxyimino-acetylamino]-3-(2 ,4-diaminopyrimidin-5-ylmethylsulfanylmethyl)-8-oxo-5-thia-1-aza-bicyclo[4, 2,0]oct-2-ene-2-carboxylic acid(23);
7-[(Z)-2-(2-amino-5-chloro-1,3-thiazol-4-yl)-2-hydroxyimino-acetylami no]-3-(2,4-diaminopyrimidin-5-ylmethylsulfanylmethyl)-8-oxo-5-thia-1-aza-bi cyclo[4,2,0]oct-2-ene-2-carboxylic acid(24).

The compounds of the formula (I) according to the present invention include syn- isomers and mixtures of syn- and anti- isomers containing 90% or more of syn- isomers. Hydrates and solvates of the compounds of the formula (I) also lie within the scope of the present invention. In addition, aminothiazole group of the compounds of the formula (I) may form tautomers with iminothiazoline group as follows:

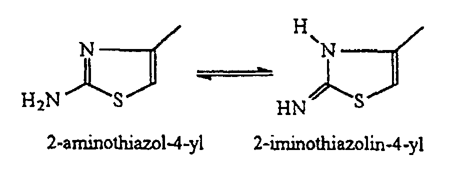

Provided that Q represents N, aminothiazole group may also form tautomers with iminothiadiazoline group as follows:

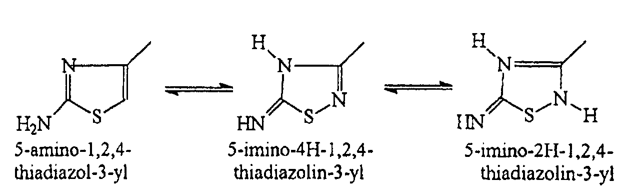

Therefore, such tautomers lie within the scope of the present invention.

Pharmaceutically acceptable nontoxic salts of the compounds of the formula (I) include salts with inorganic acid such as hydrochloric acid, hydrobromic acid, phosphoric acid or sulfuric acid, organic carboxylic acids such as acetic acid, trifluoroacetic acid, citric acid, formic acid, maleic acid, oxalic acid, succinic acid, benzoic acid, tartaric acid, fumaric acid, mandelic acid, ascorbic acid or malic acid, methane sulfonate, para-toluenesulfonate, or other acids which are conventionally used in the field of penicillin and cephalosporin. These acid addition salts may be prepared by conventional techniques. The compounds of the formula (I) may also form nontoxic salts with base. Such base includes inorganic base such as alkali metal hydroxide(e.g. sodium hydroxide, potassium hydroxide), alkali metal hydrogen carbonate(e.g. sodium hydrogen carbonate, potassium hydrogen carbonate), alkali metal carbonate(e.g. sodium carbonate, potassium carbonate, calcium carbonate), etc. or organic base such as amino acid.

Physiologically hydrolyzable esters of the compounds of the formula (I) include indanyl phthalidyl, methoxymethyl, pivaloyloxymethyl, glycyloxymethyl, phenylglycyloxymethyl, 5-methyl-2-oxo-1,3-dioxolen-4-ylmethyl and other physiologically hydrolyzable esters which are conventionally used in the field of penicillin and cephalosporin. These esters may be prepared by known methods.

The compounds of the formula (I), pharmaceutically acceptable nontoxic salts, physiologically hydrolyzable esters, hydrates and solvates thereof according to the present invention can be prepared by a process characterized in that:
(a) compounds of the following formula (V-a): 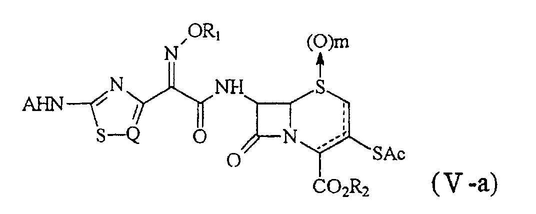 , wherein A, R₁, R₂, and Q are each as defined above and m is 0 or 1, are reacted in the presence of base with compounds of the following formula (VI-a): 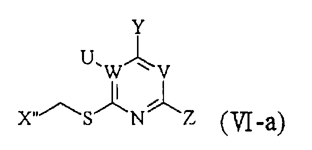 , wherein U, V, W, Y, and Z are each as defined above and X" is halogen atom, to prepare compounds of the following formula ( I -a): 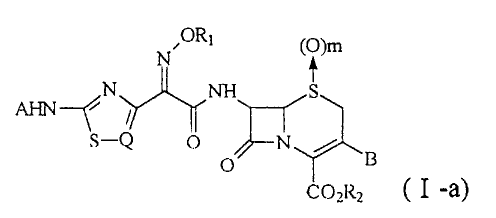 , wherein A, B, R₁, R₂, Q and m are each as defined above,
(b) compounds of the following formula (V-b): 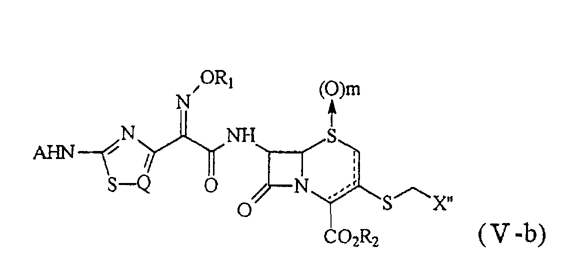 , wherein A, R₁, R₂, Q, m and X" are each as defined above, are reacted with compounds of the following formula (VI-b): 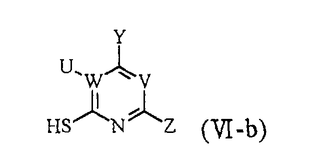 wherein U, V, W, Y, and Z are each as defined above, to prepare compounds of the above formula (I-a),
(c) compounds of the above formula (V-a) are reacted in the presence of base with compounds of the following formula (VI-c): 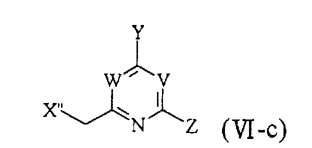 , wherein V, W, Y, Z and X" are each as defined above, or compounds of the following formula (VI-d): 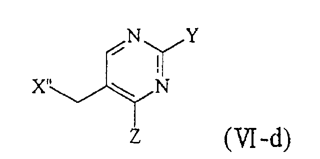 , wherein Y, Z and X" are each as defined above, to prepare compounds of the above formula ( I -a),
(d) compounds of the following formula (V-c): 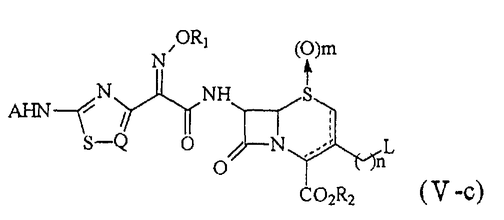 , wherein A, R₁, R₂, Q, n and m are each as defined above and L is leaving group, are reacted with in the presence of base with compounds of the following formula (VI-e): 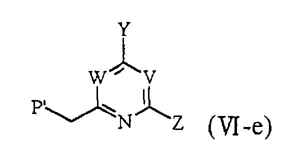 , wherein V, W, Y and Z are each as defined above and P' is thiol, alkyl- or arylthioester, or compounds of the following formula (VI -f): 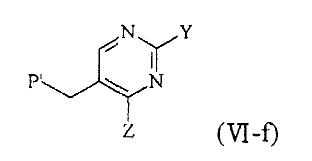 , wherein Y, Z and P' are each as defined above, to prepare compounds of the above formula ( I -a),
(e) compounds of the above formula (V-c) are reacted with compounds of the above formula (VI-b) to prepare compounds of the above formula (I-a), or
(f) compounds of the above formula (I-a), wherein m is 1, are reduced to compounds of the above formula (I).

Conventional bases in the present art, preferably, organic amine, may be used for the above-described process variants.

In the above formulae, amino protecting group A represents conventional amino protecting group such as acyl, optionally substituted ar(lower)alkyl(e.g. benzyl, diphenylmethyl, triphenylmethyl, 4-methoxybenzyl, etc.), halo(lower)alkyl(e.g. trichloromethyl, trichloroethyl, etc.), tetrahydropyranyl, substituted phenylthio, substituted alkylidene, substituted aralkylidene, substituted cyclolidene, etc. Acyl group useful as protecting group may be aliphatic or aromatic acyl group or heterocycle-containing acyl group. Examples of these acyl groups are C₁₋₅lower alkanoyl(e.g. formyl, acetyl, etc.), C₂₋₆alkoxycarbonyl(e.g. methoxycarbonyl, ethoxycarbonyl, etc.), lower alkane sulfonyl(e.g. methylsulfonyl, ethylsulfonyl, etc.) or ar(lower)alkoxycarbonyl(e.g. benzyloxycarbonyl, etc.), etc. The above-described acyl groups may contain suitable substituents such as 1∼3 halogen(s), hydroxy, cyano, nitro, etc. Other than the above described groups, compounds having amide bond with 1 or 2 amino acid(s), silane, boron or product by reaction of phosphorous compound with amino group may also be conveniently used as amino protecting group.

Carboxy protecting group R₂ which can be readily eliminated under mild condition may be conveniently used, for example, (lower)alkyl ester(e.g. methyl ester, t-butyl ester, etc.), (lower)alkenyl ester(e.g. vinyl ester, allyl ester, etc.), (lower)alkylthio(lower)alkyl ester(e.g. methylthiomethyl ester, etc.), halo(lower)alkyl ester(e.g. 2,2,2-trifluoroethyl ester, etc.), optionally substituted aralkyl ester(e.g. benzyl ester, p-nitrobenzyl ester, p-methoxybenzyl ester, etc.) or silyl ester, etc.

The amino protecting group and the carboxy protecting group should be readily eliminated under mild condition, such as hydrolysis, reduction and the like, to be converted to free amino group and carboxy group, respectively. They may be chosen and used in accordance with chemical properties of the compounds of the formula (I).

Leaving group X" represents halogen atom such as chlorine, fluorine or iodine and the like. Halogen atom represented by X" in the formulae (V-b), (VI-a), (VI-c) and (VI-d) may be converted to another halogen atom by conventional methods. For example, the compounds of the formulae (V-b), (VI-a) and (VI-c) wherein X" is iodine atom can be prepared by reaction of the compounds of the formulae (V-b), (VI-a), (VI-c) and (VI-d) wherein X" is chlorine atom with iodinated alkali metal.

Leaving group L may be halogen atom such as chlorine, fluorine and iodine, etc., (lower)alkanoyloxy such as acetoxy, etc., (lower)alkane sulfonyloxy such as methane sulfonyloxy, trifluoromethane sulfonyloxy(synthesis of the precursors thereof, 3-hydroxy compounds: Hel. Chem. Acta 1974, 57, 1919-1934), etc. arene sulfonyloxy such as para-toluene sulfonyloxy, or alkoxycarbonyloxy.

Starting materials, compounds of the formulae (V-a), (V-b) and (V-c) may be prepared by a process as shown in the following reaction scheme (I). Specifically, compounds of the following formula (VII) or salts thereof are activated with acylating agent and reacted with compounds of the following formula (VIII-a) or (VIII-b).

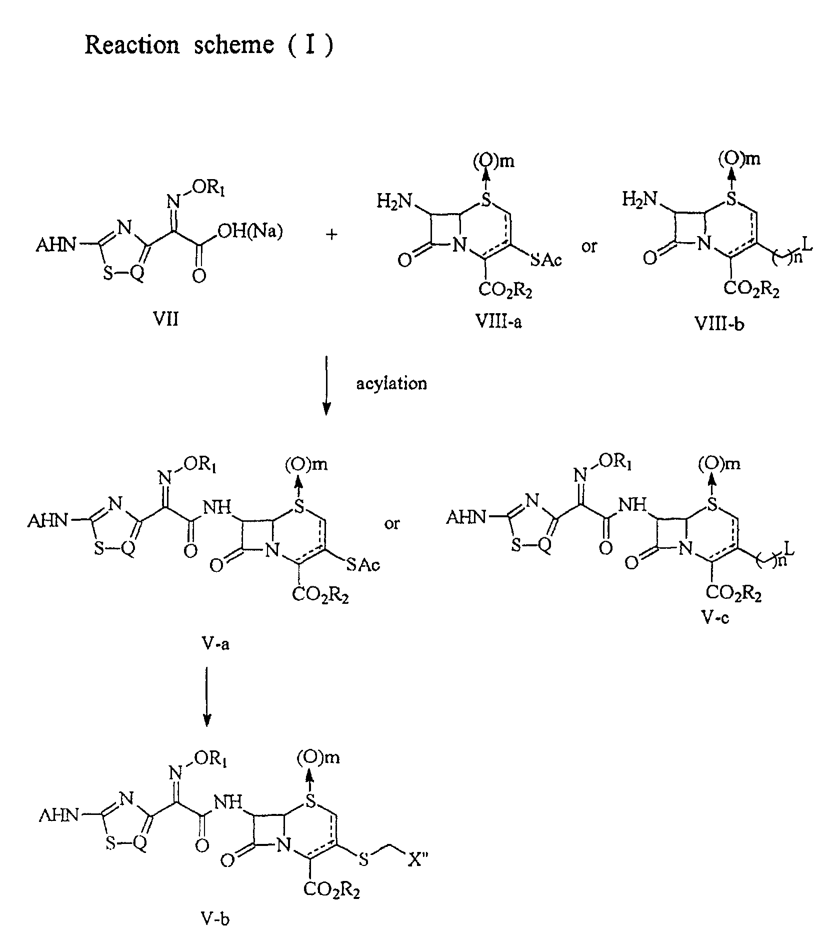

In the above scheme, A, R₁, R₂, Q, m, L, X" and n are each as defined above.

Dotted lines in the formulae (VIII-a) and (VIII-b) indicate 2-cephem compound or 3-cephem compound alone, or mixtures thereof. That is, dotted lines in the formulae (VIII-a) and (VIII-b) indicate that compounds of the formulae (VIII-a) and (VIII-b) represent compounds of the following formula (VIII-a') or (VIII-a") alone, or mixtures thereof, and compounds of the following formula **(VIII-b')** or **(VIII-b")** alone, or mixtures thereof, respectively.

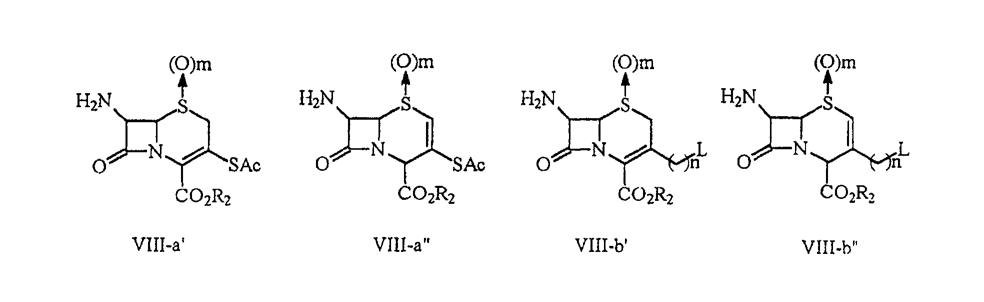

In case of preparation of compounds of the formulae (V-a) and (V-c), acylated derivatives which are activated forms of compounds of the formula (VII) may be acid chloride, acid anhydride, mixed acid anhydride(preferably, acid anhydride with methylchloroformate, mesitylenesulfonyl chloride, p-toluenesulfonyl chloride or chlorophosphate) or activated ester(preferably, ester prepared by reaction with N-hydroxybenzotriazole in the presence of condensing agent such as dicyclohexyl carbodiimide) and the like. Also, acylation reaction may be performed using compounds of the formula (VII) as free acids in the presence of condensing agent such as dicyclohexyl carbodiimide or carbonylimidazole. Acylation reaction may be preferably performed in the presence of organic base such as conventional tertiary amine(preferably, triethylamine, dimethylaniline or pyridine) or inorganic base such as sodium hydrogen carbonate or sodium carbonate. Solvents are halogenated carbon such as methylene chloride or chloroform, tetrahydrofuran, acetonitrile, dimethylformamide or dimethyl acetamide. In addition, mixtures thereof or solvents in the form of aqueous solution may be conveniently used. Acylation reaction may be performed at a temperature of -50∼50°C, preferably, -30∼20°C. And the same or slightly excess equivalents, i.e. 1.05∼1.2 equivalents, of acylating agent may be used for compounds of the formula (VII) per 1.0 equivalent of compounds of the formula (VIII-a) or (VIII-b).

Compounds of the formula (V-b) may be obtained using compounds of the formula (V-a). Specifically, compounds of the formula (V-b) may be prepared by reaction of compounds of the formula (V-a) with dihalogenomethane in the presence of base. Conventional bases in the present art, preferably, organic amine, e.g. secondary amine or aromatic amine, or dihalogenomethane, e.g. bromochloromethane or chloroiodomethane, may be conveniently used.

In case of preparation of compounds of the formula (I), amino protecting group or acid protecting group for compounds of the formulae (V-a), (V-b) and (V-c) may be eliminated by conventional methods which are well known in the field of cephalosporin. Specifically, the protecting group may be eliminated by hydrolysis or reduction. When the protecting group comprises amido group, amino halogenation or amino etherification followed by hydrolysis is preferable. Acid hydrolysis is useful for elimination of tri(di)phenylmethyl or alkoxycarbonyl group and performed using organic acid such as formic acid, trifluoroacetic acid or p-toluenesulfonic acid, or inorganic acid such as hydrochloric acid.

In a process characterized in that compounds of the formula (V -c) are reacted with compounds of the formula (VI-b), organic solvent may be used such as lower alkylnitrile such as acetonitrile or propionitrile, lower halogenated alkane such as chloromethane, dichloromethane or chloroform, tetrahydrofuran, dioxane, ether such as ethyl ether, amide such as dimethylformamide, ester such as ethyl acetate, ketone such as acetone, hydrocarbon such as benzene, alcohol such as methanol or ethanol, sulfoxide such as dimethylsulfoxide, or mixtures thereof. The process may be performed at a temperature of -80 ∼50°C, preferably, -60∼20°C. In this process, 0.5 to 2 equivalents, preferably, 1.0 to 1.1 equivalent(s), of compounds of the formula (VI-b) may be used per 1.0 equivalent of compounds of the formula (V-c).

Another process for preparation of compounds of the formula (I) is characterized by reacting compounds of the formula (VI-b) with compounds of the formula (VIII-b), wherein n is 0, to form compounds of the formula (IX), eliminating P' from amine of compounds of the formula (IX) and then, reacting compounds of the formula (IX) with compounds of the formula (X) prepared by activating carboxylic acid of compounds of the formula (VII)(processes for preparation of activated carboxylic acids and reactions thereof with amine groups: EP 94105499.1 and EP 94108809.8).

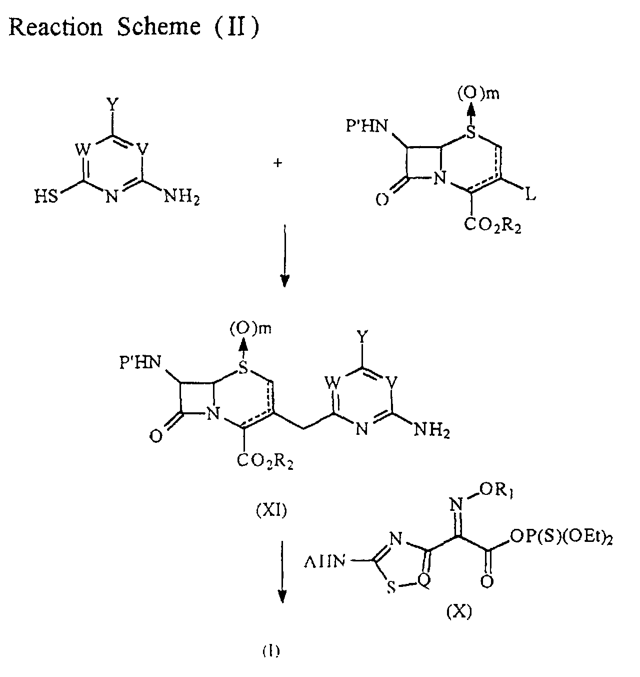

In the above scheme,
A, R₁, R₂, Q, m, P' and L are each as defined above.

Compounds of the formula (I) may be isolated or purified from products of the above-described reactions by recrystallization, electrophoresis, column chromatography on silica gel, ion-exchange chromatography and the like.

The compounds of the present invention may be administered by oral route or by injection in accordance with the purpose thereof.

Compounds of the formula (I) of the present invention may be comprised in unit dosage form or multiple dosage container by formulating them in combination with pharmaceutically acceptable carriers and excipients. A suitable dosage form is solution in oil or in aqueous medium, suspension or emulsion and comprises conventional dispersing agent, suspending agent or stabilizing agent. And dry powder may be useful which is dissolved before its use in water free from microorganisms and pyrogens. Compounds of the formula ( I ) may be formulated into suppositories using base such as cocoa butter or glyceride. Among orally administrable dosage forms, capsules, tablets, pills, powder and granules, particularly, capsules and tablets, may be useful. Preferably, tablets and pills may be enteric-coated. For solid dosage forms, compounds of the formula (I) of the present invention may be combined with carriers, for example, inert diluent such as sucrose, lactose, starch, etc. and lubricant such as magnesium stearate, disintegrating agent, binding agent and the like.

The compounds of the present invention have a great advantage of exhibiting high antimicrobial activity even in case of oral administration. This can be supported by the following test example 2, i.e. pharmacokinetic experiment on mice, which shows that blood concentrations of the present compounds were sustained for a long time in case of oral administration.

If desired, the compounds according to the present invention may be administered in combination with other antibiotics such as penicillin or other cephalosporin.

In case of formulating the compounds of the present invention into unit dosage forms, these unit dosage forms may preferably comprise about 50 to 1,500mg of the present compounds. Dose of the present compounds may be determined according to physician's prescription depending on patient's body weight, age, and characteristic property and severity of disease. However, dose for treatment of adult may be usually within the range of about 500 to 5,000mg a day in accordance with administration frequency and intensity. In case of intramuscular or intravenous injection to adult, about 150 to 3,000mg of the total dose divided into several unit doses may be appropriate a day but in case of infection by some specific bacteria, higher daily dose may be required.

The compounds of the present invention and nontoxic salts thereof, particularly, salts with alkali metal, alkali-earth metal, inorganic acid, organic acid or amino acid, exhibit potent antimicrobial activity against a wide range of pathogenic bacteria including various Gram positive and Gram negative bacteria and have broad antibacterial spectrum and therefore, are very useful for treatment or prophylaxis of diseases caused by such bacterial infection in human beings as well as animals.

### EXAMPLES

This invention will be better understood from the following examples. However, one skilled in the art will readily appreciate the specific examples described are merely illustrative of, and are not intended to, nor should be intended to, limit the invention as described more fully in the claims which follow thereafter.

### Preparation 1: Synthesis of 2-chloromethylsulfanyl-pyrimidin-4-ylamine

5g(39.32 mmol) of 4-amino-2-mercapto-pyrimidine was added to 40mℓ of DMF and the mixture was cooled down to 0°C. To this mixture was added 1.57g of sodium hydride and the whole mixture was stirred for 30 minutes. Thereto was added 40mℓ of bromochloromethane and the resulting mixture was stirred for 30 minutes. Then, this mixture was diluted with ethyl acetate and washed with saline water, and magnesium sulfate was added thereto. The resulting mixture was filtered and the solvent was removed under reduced pressure to obtain 6.2g(yield: 90%) of the title compound.
¹H NMR(CDCl₃) δ 5.20 (bros,2H) ,5.30 (s,2H), 6.20 (d,1H,5.8Hz), 8.12 (d,1H,5.9Hz)
Mass(FAB, m/e) : 175

### Preparation 2: Synthesis of 2-chloromethylsulfanyl-pyrimidine

4g(yield: 85%) of the title compound was obtained using 3g of 2-mercaptopyrimidine according to the same procedure as preparation 1.
¹H NMR(CDCl₃) δ 5.31(s,2H), 7.09(t,1H,4.6Hz), 8.63(d, 1H, 4.8Hz)
Mass(FAB, m/e) : 160

### Preparation 3: Synthesis of 2-chloromethylsulfanyl-pyrimidine-4,6-diam -ine

4.4g(yield: 66%) of the title compound was obtained using 5g of 2-mercapto-4,6-diamino-pyrimidine according to the same procedure as preparation 1.
¹H NMR(DMSOd₆) δ 5.20(s,1H), 5.41(s,2H), 6.27(bros,4H)
Mass(FAB, m/e) : 190

### Preparation 4: Synthesis of 6-chloromethylsulfanyl-pyrimidine-2,4-diam -ine

1.5g(yield: 56%) of the title compound was obtained using 2g of 2,4-diamino-6-mercapto-pyrimidine according to the same procedure as preparation 1.
¹H NMR(DMSOd₆) δ 5.43(s, 2H), 5.76(s, 1H), 6.20(bros, 2H), 6.60(bros, 2H)
Mass(FAB, m/e) : 190

### Preparation 5: Synthesis of 3-acetylsulfanyl-7-{2-methoxyimino-2-[2-(tri -tyl-amino)-thiazol-4-yl]-acetylamino}-8-oxo-5-thia-1-aza-bicyclo[4,2,0]oct-2-ene-2-carboxylic acid benzhydryl ester

1.67g(3.564 mmol) of (Z)-methoxyimino-[2-(trityl-amino)-thiazol-4 -yl]-acetic acid and 1.7g(1.0 equivalent) of 3-acetylsulfanyl-7-amino-8-oxo -5-thia-1-aza-bicyclo[4,2,0]oct-2-ene-2-carboxylic acid benzhydryl ester were added to 50mℓ of methylene chloride and the mixture was cooled down to -30°C. To this mixture were added 0.72mℓ of pyridine and 0.35mℓ of phosphorous oxychloride. The whole mixture was stirred for 2 hours, diluted with ethyl acetate and washed with saline water. Magnesium sulfate was added thereto. This mixture was filtered and the solvent was removed under reduced pressure. The residue was purified by column chromatography to obtain 2.5g(yield: 80%) of the title compound.
¹H NMR(CDCl₃) δ 2.02(s,3H), 3.38(d,1H,17.2Hz), 3.82(d,1H,17.4Hz), 4.05(s,3H), 5.18(d,1H,4.5Hz), 6.00(m,1H), 6.70(s,1H), 7.00(s,1H), 7.20 ∼ 7.44(m.25H)
Mass(FAB, m/e) : 865

### Preparation 6: Synthesis of 3-acetylsulfanyl-7-{2-[5-chloro-2-(trityl-amin o)-thiazol-4-yl]-2-methoxyimino-acetylamino}-8-oxo-5-thia-1-aza-bicyclo[4, 2,0]oct-2-ene-2-carboxylic acid

2g(yield: 53%) of the title compound was obtained using 2.1g of (Z)-[5-chloro-2-(trityl-amino)-thiazol-4-yl]-methoxyimino acetic acid according to the same procedure as preparation 5.
¹H NMR(CDCl₃) δ 2.14(s,3H), 3.33(d,1H,17.2Hz), 3.80(d,1H,17.1Hz), 4.06(s,3H), 5.17(d,1H,4.4Hz), 6.03(m,1H), 6.99(s,1H), 7.22 ∼ 7.40(m.25H)
Mass(FAB, m/e) : 899

### Preparation 7: Synthesis of 3-acetylsulfanyl-7-[2-(2-t-butoxycarbonylam ino-thiazol-4-yl)-2-trityloxyimino-acetylamino]-8-oxo-5-thia-1-aza-bicyclo] 4,2,0]oct-2-ene-2-carboxylic acid benzhydryl ester

2.5g(yield: 70%) of the title compound was obtained using 2g of (Z)-(2-t-butoxycarbonylamino-thiazol-4-yl)-trityloxyimino-acetic acid accord -ing to the same procedure as preparation 5.
¹H NMR (CDCl₃) δ 1.49 (s,9H), 2.07 (s,3H), 3.27 (d,1H,17.5Hz), 3.76 (d,1H,17.2Hz), 5.16 (d,1H,4.4Hz), 6.08 (m,1H), 6.98 (s,1H), 7.01 (s,1H), 7.20 ∼ 7.40(m.25H)
Mass(FAB, m/e) : 951

### Preparation 8: Synthesis of 3-acetylsulfanyl-7-[2-(2-t-butoxycarbonylam ino-5-chloro-thiazol-4-yl)-2-trityloxyimino-acetylamino]-8-oxo-5-thia-1-aza -bicyclo[4,2,0]oct-2-ene-2-carboxylic acid benzhydryl ester

2.2g(yield: 63%) of the title compound was obtained using 2g of (Z)-(2-t-butoxycarbonylamino-5-chloro-thiazol-4-yl)-trityloxyimino-acetic acid according to the same procedure as preparation 5.
¹H NMR (CDCl₃) δ 1.49 (s,9H), 2.04 (s,3H), 3.27 (d,1H,17.2Hz), 3.78 (d,1H,17.4Hz), 5.14 (d,1H,4.4Hz), 6.02(m,1H), 7.01(s,1H), 7.21 ∼ 7.41(m.25H)
Mass(FAB, m/e) : 981

### Preparation 9: Synthesis of 7-[2-(2-t-butoxycarbonylamino-5-chloro-thia zol-4-yl)-2-trityloxyimino-acetylamino]-3-chloromethylsulfanyl-8-oxo-5-thi a-1-aza-bicyclo[4,2,0]oct-2-ene-2-carboxylic acid benzhydryl ester

2.39g (2.47mmol) of 3-acetylsulfanyl-7-[2-(2-t-butoxycarbonylamino -5-chloro-thiazol-4-yl)-2-trityloxyimino-acetylamino]-8-oxo-5-thia-1-aza-bicyc lo[4,2,0]oct-2-ene-2-carboxylic acid benzhydryl ester was dissolved in 20 mℓ of DMF and the solution was cooled down to -5°C. To this solution was added 2.4mℓ of aliquots of solution prepared by mixing 0.44mℓ of morpholine and 0.41mℓ of pyridine followed by diluting the mixture with benzene to adjust its volume to 10mℓ and the whole mixture was stirred for 30 minutes. 360µℓ(2.0 equivalents) of chloroiodomethane was added thereto and the resulting mixture was warmed up to 10°C. This mixture was stirred for 2 hours and diluted with ethyl acetate. The resulting mixture was washed with 1% of hydrochloric acid solution and saline water and the solvent was removed under reduced pressure. The residue was purified by column chromatography to obtain 1.72g(yield: 70%) of the title compound.
¹H NMR (CDCl₃) δ 1.55 (s, 9H), 3.55 (d, 1H, 17.4Hz), 3.75 (d, 1H, 17.3Hz), 4.60 (d, 1H, 12.4Hz), 4.73 (d, 1H, 12.3Hz), 5.10 (d, 1H, 4.6Hz), 6.02 (m, 1H), 6.96 (s, 1H), 7.25∼7.35(m, 30H), 8.22 (bros, 1H)
Mass (FAB, m/e): 992

### Preparation 10: Synthesis of 7-[(Z)-2-(2-t-butoxycarbonylamino-5-chlor o-thiazol-4-yl)-2-trityloxyimino-acetylamino]-8-oxo-3-(1-chloromethylthio) -5-thia-1-aza-bicyclo[4,2,0]oct-2-ene-2-carboxylic acid benzhydryl ester

4g(4.13mmol) of 3-acetylsulfanyl-7-[(Z)-2-(2-t-butoxycarbonylamino -thiazole-5-chloro-4-yl)-2-trityloxyimino-acetylamino]-8-oxo-5-thia-1-aza-bicy clo[4,2,0]oct-2-ene-2-carboxylic acid benzhydryl ester was dissolved in 40 mℓ of DMF and the solution was cooled down to 0°C. 4.13mℓ of aliquots of solution prepared by dissolving 83mℓ of triethyl amine and 50 mℓ of morpholine in 467mℓ of benzene was added to this solution and the resulting mixture was stirred for 30 minutes. 0.54mℓ of bromochloromethane was added thereto and the mixture was stirred overnight at 5°C. This mixture was diluted with ethyl acetate and washed with saline water and then, the solvent was removed under reduced pressure. The residue was purified by column chromatography to obtain 1.7g(yield: 42%) of the title compound.
¹H NMR(CDCl₃) δ 1.49(s, 9H), 3.56(d, 1H, 16.9Hz), 3.77(d, 1H, 16.8Hz), 4.60(d, 1H, 11.2Hz), 4.76(d, 1H, 11.2Hz), 5.20(d, 1H, 4.9Hz), 6.00(m, 1H), 6.96(s, 1H), 7.26∼7.35(m, 25H), 8.11(br, s, 1H)
Mass(FAB, m/e): 991

### Preparation 11: Synthesis of 7-[(Z)-2-(2-t-butoxycarbonylamino-5-chloro-thiazol-4-yl)-2-trityloxyimino-acetylamino]-8-oxo-3-(1-chloroethyl thio)-5-thia-1-aza-bicyclo[4,2,0]oct-2-ene-2-carboxylic acid benzhydryl es ter

2g(2mmol) of 3-acetylsulfanyl-7-[(Z)-2-(2-t-butoxycarbonylaminothia -zole-5-chloro-4-yl)-2-trityloxyimino-acetylamino]-8-oxo-5-thia-1-aza-bicyclo[ 4,2,0]oct-2-ene-2-carboxylic acid benzhydryl ester was dissolved in 20mℓ of DMF and the solution was cooled down to 0°C. To this solution was added 2.06mℓ of aliquots of solution prepared by dissolving 83mℓ of triethylamine and 50mℓ of morpholine in 467mℓ of benzene and the resulting mixture was stirred for 30 minutes. 0.5mℓ of bromochloroethane was added thereto and the whole mixture was stirred overnight at 5°C. This mixture was diluted with ethyl acetate and washed with saline water and then, the solvent was removed under reduced pressure. The residue was purified by column chromatography to obtain 0.8g(yield: 40%) of the title compound.
¹H NMR(CDCl₃) δ 1.58(s, 9H), 2.94(m, 2H), 3.32(d, 1H, 17.0Hz)_{,} 3.44(m, 3H), 5.09(d, 1H, 4.6Hz), 5.94(m, 1H), 6.97(s, 1H), 7.28∼7.36(m, 25H), 8.20(br, s, 1H)
Mass(FAB, m/e): 1005

### Preparation 12: Synthesis of 3-(acetylsulfanyl)-7-{[2-[2-({(2S)-2-[(t-butox ycarbonyl)amino]-2-phenylacetyl}amino)-5-chloro-1,3-thiazol-4-yl]-2-(met hoxyimino)acetyl]amino}-8-oxo-5-thia-1-aza-bicyclo[4,2,0]oct-2-ene-2-carb oxylic acid benzhydryl ester

3.1g (7.742 mmol) of 2-[2-({(2S)-2-[(t-butoxycarbonyl)amino]-2-phe -nylacetyl}amino)-5-chloro-1,3-thiazol-4-yl]-2-(methoxyimino)acetic acid ethyl ester was dissolved in THF/H₂O(30mℓ/30mℓ) solvent. 974mg(23.2245 mmol) of lithium hydroxide monohydrate was added thereto and the mixture was stirred for 10 hours at room temperature. This mixture was distilled under reduced pressure to remove THF and the residue was acidified with 1N of HCl solution to a pH of 3. This residue was extracted with ethyl acetate and the organic layer was dried over magnesium sulfate and distilled under reduced pressure. The obtained solid compound was used in the subsequent reaction without further purification.

900mg(1.9196 mmol) of the obtained compound and 914mg (1.9196 mmol) of 3-(acetylsulfanyl)-7-amino-8-oxo-5-thia-1-aza-bicyclo[4,2, 0]oct-2-ene-2-carboxylic acid benzhydryl ester were dissolved in 15mℓ of dichloromethane. 390µℓ(4,799 mmol) of pyridine was added thereto and the mixture was cooled down to -30°C. 233µℓ(2,4955 mmol) of phosphoryl oxychloride was added thereto. The mixture was warmed up to -10°C and stirred for 2 hours. Excess ethyl acetate was added thereto and the whole mixture was washed with water and saline water. The organic layer was dried over magnesium sulfate and then, filtered and distilled under reduced pressure. The residue was purified by column chromatography to obtain the title compound(yield: 60.4%).
¹H NMR(CDCl₃)δ 10.81(br, s, 1H), 7.89(br, s, 1H), 7.35∼ 7.25(m, 15H), 7.00(s, 1H), 5.99∼5.98(d, 1H, J=5.04Hz), 5.18(br, m, 1H), 5.22∼5.21(d, 1H, J=5.04Hz), 4.07(s, 3H), 3.89∼3.81(Abq, 1H, J=17.87Hz), 3.45∼3.43(Abq, 1H, J=17.87Hz), 2.01(s, 3H), 1.43(s, 9H)
Mass(m/e): 819

### Preparation 13: Synthesis of 2-[2-({(2S)-2-[(t-butoxycarbonyl)amino]-2-phenylacetyl}amino)-5-chloro-1,3-thiazol-4-yl]-2-(methoxyimino)acetic aci d ethyl ester

100mg(0.4363 mmol) of 2-(2-amino-5-chloro-1,3-thiazol-4-yl)-2-(me -thoxyimino)acetic acid ethyl ester and 83mg (0.4363mmol) of butoxycarbonyl-(L)-phenylglycol were dissolved in 3mℓ of DMF. 59mg(0.4363 mmol) of HOBT(hydroxybenzotriazole), 100mg(0.5236 mmol) of EDC (1-ethyl-3-(3-dimethylaminopropyl)carbodiimide) were subsequently added thereto and the whole mixture was stirred for 10 hours at room temperature. Excess ethyl acetate was added thereto and the resulting mixture was washed with water and saline water. The organic layer was dried over magnesium sulfate and then, filtered and distilled under reduced pressure. The residue was purified by column chromatography to obtain the title compound(yield: 88.7%).
¹H NMR(CDCl₃) δ 9.72(br, m, 1H), 7.33∼7.32(m, 5H), 5.54(br, m, 1H), 4.35∼4.33(q, 2H), 4.00(s 3H), 1.41(s, 9H), 1.34∼1.31(t, 3H)
Mass(m/e): 496

### Preparation 14: Synthesis of 2,4-diamino 5-carbethoxy pyrimidine

5.88g(27.6 mmol) of 4-amino 5-carbethoxy 2-thiomethylpyrimidine was dissolved in 60mℓ of dichloromethane and the solution was cooled down to 0°C. To this solution was slowly added dropwise 11.4g(1.2 equivalents, ∼50%) of metachloroperbenzoic acid dissolved in 40mℓ of dichloromethane. The reaction was performed for 2 hours at 0°C and then, stopped with 5% of sodium thiosulfate solution. The reaction mixture was washed with sodium hydrogen carbonate solution and concentrated by distillation under reduced pressure. To this concentrate was added 100mℓ of 28% aqueous ammonium solution and the mixture was stirred for 2 hours at room temperature. This mixture was neutralized with acetic acid and then, filtered and dried to obtain 2g(yield: 42%) of the title compound as solid.
¹H NMR(DMSO) δ 1.25(t, 3H), 4.19(q, 2H), 7.23(s, 1H), 7.38(s, 1H), 7.45(s, 1H)
Mass(FAB, m/e) : 183

### Preparation 15: Synthesis of 2,4-diamino-5-hydroxymethyl pyrimidine

1.2g(6.6 mmol) of 2,4-diamino-5-hydroxymethylpyrimidine was suspended in 1,1,1,3,3,3-hexamethyldisilazane. To this suspension was added a catalytic amount of ammonium sulfate and the mixture was refluxed. After 24 hours, hexamethyldisilazane was removed under reduced pressure and the residue was dissolved in anhydrous tetrahydrofuran and the solution was cooled down to 0°C . 250mg(1.1 equivalents) of lithium aluminum hydride was added dropwise thereto and the mixture was warmed up to room temperature. The reaction was performed for 30 minutes and then, stopped with water and acetone. The reaction mixture was filtered and the solvent was removed under reduced pressure to obtain 830mg of the title compound.
¹H NMR(DMSO) δ 4.19(s, 2H), 5.79(s, 2H), 6.07(s, 2H), 7.57(s, 1H)
Mass(FAB, m/e): 140

### Preparation 16: Synthesis of 2,4-diamino-5-bromomethyl pyrimidine hydrobromide

165mg(1.178 mmol) of 2,4-diamino-5-hydroxymethyl pyrimidine was dissolved in 9mℓ of acetic acid and the solution was cooled down to 5°C. To this solution was added dropwise 2.5mℓ of hydrobromic acid(in 30% of acetic acid solution). The reaction was performed for 30 minutes at room temperature and the reaction mixture was warmed up to 60°C. The reaction was further performed for 2 hours and then, the solvent was removed under reduced pressure. The residue was dissolved in a small amount of ethanol and ether was added thereto. The resulting mixture was filtered and dried to obtain 408mg of the title compound.
¹H NMR(D₂O) δ 4.48(s, 2H), 7.68(s, 1H)

### Preparation 17: Synthesis of 2,4-diphenylmethylamino-6-carboxypyrimi dine diphenylmethylamine salt

450mg(2.33 mmol) of 2,4-dichloro-6-carboxypyrimidine and 5 equivalents of diphenylmethylamine were dissolved in 30mℓ of ethanol and the solution was refluxed. The reaction was performed for 24 hours and the reaction mixture was cooled down to room temperature. The mixture was filtered to obtain 710mg(yield: 45%) of the title compound as solid.
¹H NMR(DMSO) δ 5.63(s, 1H), 6.20(d, 1H), 6.38(d, 1H), 6.60(s, 1H), 7.25-7.35(m, 30H), 8.83(d, 1H), 9.58(d, 1H)

### Preparation 18: Synthesis of 2,4-diphenylmethylamino-6-hydroxymethyl pyrimidine

710mg(1.06 mmol) of 2,4-diphenylmethylamino-6-carboxypyrimidine diphenylmethylamine salt was dissolved in anhydrous tetrahydrofuran and 1.1 equivalents of lithium aluminum hydride was added dropwise thereto. The reaction was performed for 3 hours and stopped with water and acetone. The reaction mixture were filtered and the solvent was removed under reduced pressure to obtain 420mg of the title compound.
¹H NMR(DMSO) δ 4.13(s, 2H), 5.63(s, 1H)

### Preparation 19: Synthesis of 2,4-diamino-6-bromomethyl pyrimidine h ydrobromide

840mg(1.78 mmol) of 2,4-diamino-6-hydroxymethyl pyrimidine was dissolved in 12mℓ of acetic acid and the solution was cooled down to 5 °C. Thereto was added dropwise 3mℓ of hydrobromic acid(in 30% of acetic acid solution). The reaction was performed for 30 minutes and the reaction mixture was warmed up to 70 °C. This reaction was further performed for 3 hours and the solvent was removed under reduced pressure. The residue was dissolved in a small amount of ethanol an then, ether was added thereto. The resulting mixture was filtered and dried to obtain 500mg of the title compound as solid.
¹H NMR(D₂O) δ 4.38(s, 2H), 6.33(s, 1H).

### Preparation 20: Synthesis of 7-amino-3-(2,6-diamino-pyrimidin-4-ylsulfa nyl)-8-oxo-5-thia-1-aza-bicyclo[4,2,0]oct-2-ene-2-carboxylic acid

980mg(2.25 mmol) of 7-t-butyloxylcarbonylamino-3-hydroxy-8-oxo-5 -thia-1-aza-bicyclo[4,2,0]oct-2-ene-2-carboxylic acid 4-methoxybenzyl ester was dissolved in 20mℓ of methylene chloride and the solution was cooled down to -78°C. 391µℓ of diisopropylethylamine and 378µℓ of trifluoroacetic anhydride were added thereto. The mixture was stirred for 30 minutes, diluted with ethyl acetate and washed with saline water,. Magnesium sulfate was added thereto. The resulting mixture was filtered and the solvent was removed under reduced pressure. The residue was dissolved in 6mℓ of N,N-dimethylformamide and 2mℓ of tetrahydrofuran and the solution was cooled down to -78°C. Then, 448mg of 2,6-diamino-4-thiopyrimidine was added thereto and the resulting mixture was slowly warmed up to room temperature while stirring overnight. This mixture was diluted with ethyl acetate and washed with saline water and magnesium sulfate was added thereto. The resulting mixture was filtered and the solvent was removed under reduced pressure, Solid obtained by addition of methylene chloride and diethyl ether to the residue was dissolved in 1mℓ of anisole and thereto was added 2mℓ of trifluoroacetic acid. This mixture was stirred for 30 minutes and cooled down to 0°C. 10mℓ of diethylether was added thereto and the resulting mixture was filtered to obtain 540mg(yield: 70%) of the title compound.
¹H NMR(DMSOd₆) δ 3,66(d, 1H, 7.5Hz), 3.92(d, 1H, 7.5Hz), 5.15(m, 1H), 5.26(m, 1H), 5.89(m, 1H), 7.41(m, 2H), 7.72(m, 2H)
Mass(FAB, m/e): 340

### Preparation 21: Synthesis of benzhydryl 7-amino-3-[(2-amino-6-hydrox y-4-pyrimidinyl)sulfanyl]-8-oxo-5-thia-1-aza-bicyclo[4,2,0]oct-2-ene-2-carb oxylic acid

600mg(1.21 mmol) of benzhydryl 7-amino-3-[(methylsulfonyl)oxy] -8-oxo-5-thia-1-aza-bicyclo[4.2.0]oct-2-ene-2-carboxylic acid was dissolved in THF/DMF(2mℓ/6mℓ) and the solution was cooled down to -50°C. To this solution was added 172.8mg(1.21 mmol) of 2-amino-4-hydroxy-6-mercaptopyrimidine and the mixture was slowly warmed up to room temperature while stirring for 8 hours. The resulting mixture was distilled under reduced pressure to remove the solvent. Excess ether was added to the residue to obtain the title compound as solid(yield: 85%).
¹H NMR(DMSO) δ 10.95(br, s, 1H), 9.32(br, s, 1H), 7.45-7.27(m, 10H), 7.01(s, 1H), 5.40-5.39(d, 1H, J=5.05Hz), 5.34(s, 1H)_{,} 5.29-5.28(d, 1H, J=5.05Hz), 4.04-4.00(Abq, 1H, J=17.85Hz), 3.73-3.69(Abq, 1H, J=17.9Hz)
Mass(m/e) 507.6

### Preparation 22: Synthesis of benzhydryl 7-amino-3-[(2,6-diamino-pyrim idin-4-ylsulfanyl]-8-oxo-5-thia-1-aza-bicyclo[4,2,0]oct-2-ene-2-carboxylic a cid

The title compound(yield: 83%) was obtained using 2.0g(4.025 mmol) of benzhydryl 7-amino-3-[(methylsulfonyl)oxy]-8-oxo-5-thia-1-aza -bicyclo[4,2,0]oct-2-ene-2-carboxylic acid and 731mg(3.823 mmol) of 2,4-diamino-6-mercaptopyrimidine according to the same procedure as preparation 21.
¹H NMR(DMSO) δ 7.37-7.29(m, 10H), 6.94(s, 1H), 6.14(s, 1H), 5.50-5.49(d, 1H, J=5.5Hz), 5.29-5.28(d, 1H, J=5.5Hz), 4.10-4.07(Abq, 1H, J=17.85Hz), 3.78-3.75(Abq, 1H, J=17.9Hz)
Mass(m/e) 506

### Example 1: Synthesis of 7-[(Z)-2-(2-amino-thiazol-4-yl)-2-methoxyimino -acetylamino]-8-oxo-3-(pyrimidin-2-ylsulfanylmethylsulfanyl)-5-thia-1-aza -bicyclo[4,2,0]oct-2-ene-2-carboxylic acid

600mg(0.673 mmol) of 3-acetylsulfanyl-7-{2-methoxyimino-2-[2-(tri -tylamino)-thiazol-4-yl]-acetylamino}-8-oxo-5-thia-1-aza-bicyclo[4,2,0]oct-2-e ne-2-carboxylic acid benzhydryl ester was dissolved in 6mℓ of DMF and the solution was cooled down to 0°C. To this solution was added 2mℓ of aliquots of solution prepared by dissolving 0.467mℓ of triethylamine and 0.293mℓ of morpholine in 10mℓ of benzene and this mixture (A) was stirred for 30 minutes.

204mg(1.2 equivalents) of 2-chloromethylsulfanyl pyrimidine was dissolved in 2mℓ of acetone and 400mg of sodium iodide was added thereto. The mixture was stirred for 3 hours at 40∼50°C and diluted with ethyl acetate and washed with water. Magnesium sulfate was added thereto. The mixture was filtered and the solvent was removed under reduced pressure to obtain 2-iodolethylsulfanyl pyrimidine.

The obtained 2-iodolethylsulfanyl pyrimidine was added to mixture (A) and the whole mixture was stirred for 30 minutes. Then, this mixture was diluted with ethyl acetate and washed with saline water. The resulting mixture was filtered and the solvent was removed under reduced pressure. Solid obtained by addition of diethyl ether to the residue followed by filtration was dissolved in 1.5mℓ of anisole. To this solution was added 1mℓ of trifluoroacetic acid and the mixture was stirred for 60 minutes. This mixture was cooled down to 0°C and solid obtained by addition of 10mℓ of diethyl ether to the mixture followed by filtration was purified by preparative high pressure liquid chromatography to obtain 100mg(yield: 21%) of the title compound.
¹H NMR(D₂O) δ 3.70(d,1H, 17.6Hz), 3.98(d,1H, 17.2Hz), 4.02(s,3H), 4.56(s, 2H), 5.26(d,1H, 4.8Hz), 5.81(d,1H, 4.9Hz), 7.04(s,1H), 7.28(t, 1H,4.3Hz)
Mass(FAB, m/e) : 539

### Example 2: Synthesis of 7-[(Z)-2-(2-amino-thiazol-4-yl)-2-methoxyimino -acetylamino]-8-oxo-3-(4-amino-pyrimidin-2-ylsulfanylmethylsulfanyl)-5-t hia-1-aza-bicyclo[4,2,0]oct-2-ene-2-carboxylic acid

85mg(yield: 22%) of the title compound was obtained using 220mg of 2-chloromethylsulfanyl-pyrimidin-4-ylamine according to the same procedure as example 1.
¹H NMR(D₂O) δ 3.65(d,1H, 17.2Hz), 3.88(d,1H, 17.4Hz), 4.02(s,3H), 4.50(s,2H), 5.22(d,1H, 4.6Hz), 5.80(d,1H,4.7Hz), 6.34(d,1H,5.8Hz), 7.02(s,1H), 7.94(d,1H,5.9Hz)
Mass(FAB, m/e) : 554

### Example 3: Synthesis of 7-[(Z)-2-(2-amino-thiazol-4-yl)-2-methoxyimino -acetylamino]-3-(4,6-diamino-pyrimidin-2-ylsulfanylmethylsulfanyl)-8-oxo-5-thia-1-aza-bicyclo[4,2,0]oct-2-ene-2-carboxylic acid

220mg(yield: 19%) of the title compound was obtained using 750mg of 2-chloromethylsulfanyl-pyrimidine-4,6-diamine according to the same procedure as example 1.
¹H NMR(D₂O) δ 3.58(d, 1H, 17.3Hz), 3.78(d, 1H, 17.4Hz), 3.93(s,3H), 4.42(q, 2H, 14.1Hz), 5.10(d, 1H, 4.5Hz),5.40(s, 1H), 5.71(d, 1H, 4.6Hz), 6.95(s, 1H)
Mass(FAB, m/e) : 569

### Example 4: Synthesis of 7-[(Z)-2-(2-amino-thiazol-4-yl)-2-methoxyimino -acetylamino]-3-(2,6-diamino-pyrimidin-4-ylsulfanylmethylsulfanyl)-8-oxo-5-thia-1-aza-bicyclo[4,2,0]oct-2-ene-2-carboxylic acid

200mg(yield: 26%) of the title compound was obtained using 500mg of 4-chloromethylsulfanyl-pyrimidine-2,6-diamine according to the same procedure as example 1.
¹H NMR(D₂O) δ 3.58(d,1H, 17.2Hz), 3.84(d,1H, 17.1Hz), 3.98(s,3H), 4.38(q,2H,14.3Hz), 5.18(d,1H, 4.6Hz),5.79(d,1H,4.5Hz), 6.04(s,1H), 7.00(s,1H)
Mass(FAB, m/e) : 569

### Example 5: Synthesis of 7-[(Z)-2-(2-amino-thiazol-4-yl)-2-hydroxyimino-acetylamino]-3-(4,6-diamino-pyrimidin-2-ylsulfanylmethylsulfanyl)-8-oxo-5-thia-1-aza-bicyclo[4,2,0]oct-2-ene-2-carboxylic acid

640mg(0.673 mmol) of 3-acetylsulfanyl-7-[2-(2-t-butoxycarbonylami -nothiazol-4-yl)-2-trityloxyimino-acetylamino]-8-oxo-5-thia-1-aza-bicyclo[4,2, 0]oct-2-ene-2-carboxylic acid benzhydryl ester was dissolved in 6mℓ of DMF and the solution was cooled down to 0°C. To this solution was added 2mℓ of aliquots of solution prepared by dissolving 0.467mℓ of triethylamine and 0.293mℓ of morpholine in 10mℓ of benzene and this mixture (A) was stirred for 30 minutes.

230mg of 2-chloromethylsulfanyl pyrimidine-4,6-diamine was dissolved in 3mℓ of acetone and 460mg of sodium iodide was added thereto. Then, the mixture was stirred for 3 hours at 40 ∼ 50°C and diluted with ethyl acetate and washed with water. Magnesium sulfate was added thereto. The resulting mixture was filtered and the solvent was removed under reduced pressure to obtain 2-iodomethylsulfanyl pyrimidine-4,6-diamine.

The obtained 2-iodomethylsulfanyl pyrimidine-4,6-diamine was added to mixture (A). The whole mixture was stirred for 30 minutes. Then, this mixture was diluted with ethyl acetate and washed with saline water. The solvent was removed under reduced pressure. Solid obtained by addition of diethyl ether to the residue followed by filtration was dissolved in 0.5mℓ of anisole and 1mℓ of trifluoroacetic acid was added thereto. The resulting mixture was stirred for 2 hours and cooled down to 0°C. Solid obtained by addition of 10mℓ of diethyl ether to the mixture followed by filtration was purified by preparative high pressure liquid chromatography to obtain 30mg(yield: 8%) of the title compound.
¹H NMR(D₂O) δ 3.57(d,1H, 17.0Hz), 3.80(d,1H, 17.1Hz), 4.42(q, 2H,14.4Hz), 5.11(d,1H, 4.6Hz), 5.40(s,1H), 5.74(d,1H, 4.4Hz), 6.91(s,1H)
Mass(FAB, m/e) : 555

### Example 6: Synthesis of 7-[(Z)-2-(2-amino-thiazol-4-yl)-2-hydroxyimino-acetylamino]-3-(2,6-diamino-pyrimidin-4-ylsulfanylmethylsulfanyl)-8-oxo-5-thia-1-aza-bicyclo[4,2,0]oct-2-ene-2-carboxylic acid

40mg of the title compound was obtained using 240mg of 4-chloromethylsulfanyl-pyrimidine-2,6-diamine according to the same procedure as example 5.
¹H NMR (D₂O) δ 3.57 (d,1H, 17.3Hz), 3.83 (d,1H, 17.4Hz), 4.42 (q,2H,14.4Hz), 5.17(d,1H, 4.6Hz),5.81(d,1H,4.5Hz), 6.03(s,1H), 6.96(s,1H)
Mass(FAB, m/e) : 555

### Example 7: Synthesis of 7-[(Z)-2-(2-amino-5-chloro-thiazol-4-yl)-2-hydr oxyimino-acetylamino]-3-(2,6-diamino-pyrimidin-4-ylsulfanylmethylsulfan yl)-8-oxo-5-thia-1-aza-bicyclo[4,2,0]oct-2-ene-2-carboxylic acid

### (Process 1)

57mg(yield: 14%) of the title compound was obtained using 700mg of 3-acetylsulfanyl-7-[2-(2-t-butoxycarbonylamino-5-chloro-thiazol-4 -yl)-2-trityloxyimmo-acetylammo]-8-oxo-5-thia-1-aza-bicyclo[4,2,0]oct-2-ene-2-carboxylic acid benzhydryl ester and 250mg of 4-chloromethylsulfanyl-pyrimidine-2,6-diamine according to the same procedure as example 5.

### (Process 2)

1.72g(1.73 mmol) of 7-[2-(2-t-butoxycarbonylamino-5-chloro-thiazol -4-yl)-2-trityloxyimino-acetylamino]-8-oxo-3-(1-chloromethylthio)-5-thia-1-az a-bicyclo[4,2,0]oct-2-ene-2-carboxylic acid benzhydryl ester prepared in preparation 9 was dissolved 20mℓ of DMF. 740mg of sodium iodide and 567mg of 2.4-diamino-6-mercaptopyrimidine hemisulfate were added to this solution. The resulting mixture was stirred for 20 hours and then, diluted with ethyl acetate and washed with water. The solvent was removed under reduced pressure. Solid obtained by addition of diethyl ether to the residue followed by filtration was dissolved in 2mℓ of anisole. This solution was cooled down to 0°C and 4mℓ of trifluoroacetic acid was added thereto. The resulting mixture was warmed up to room temperature and stirred for 3 hours and then, cooled down to 0°C again. Solid obtained by addition of 25mℓ of diethyl ether to the mixture followed by filtration was purified by preparative high pressure liquid chromatography to obtain 200mg(yield: 20%) of the title compound.
¹H NMR(D₂O) δ 3.60 (d,1H, 17.4Hz), 3.86 (d,1H, 17.3Hz), 4.42 (q,2H,25Hz), 5.21 (d,1H, 4.6Hz), 5.87 (d,1H,4.6Hz), 6.04 (s,1H)
Mass(FAB, m/e) : 589

### Example 8: Synthesis of 7-[(Z)-2-(2-amino-5-chloro-thiazol-4-yl)-2-meth oxyimino-acetylamino]-3-(2,6-diamino-pyrimidin-4-ylsulfanylmethylsulfan yl)-8-oxo-5-thia-1-aza-bicyclo[4,2,0]oct-2-ene-2-carboxylic acid

260mg(22%) of the title compound was obtained using 1.4g of 3-acetylsulfanyl-7-{2-methoxyimino-2-[2-(tritylamino)-5-chloro-thiazol-4-yl]-a cetylamino}-8-oxo-5-thia-1-aza-bicyclo[4,2,0]oct-2-ene-2-carboxylic acid benzhydryl ester and 520mg of 4-chloromethylsulfanyl-pyrimidine-2,6-diam -ine according to the same procedure as example 1.
¹H NMR(D₂O) δ 3.57(d, 1H, 17.2Hz), 3.85(d, 1H, 17.3Hz), 4.04(s, 3H), 4.38(q, 2H, 18.5Hz), 5.19(d, 1H, 4.6Hz), 5.82(d, 1H, 4.5Hz), 6.01(s, 1H)
Mass(FAB, m/e) : 589

### Example 9: Synthesis of 7-[(Z)-2-(2-amino-thiazol-5-chloro-4-yl)-2-hydr oxyimino-acetylamino]-3-(2-amino-6-methyl-pyrimidin-4-ylsulfanylmethyl sulfanyl)-8-oxo-5-thia-1-aza-bicyclo[4,2,0]oct-2-ene-2-carboxylic acid

1.45g(1.5 mmol) of 3-acetylsulfanyl-7-[(Z)-2-(2-t-butoxycarbonylami -nothiazole-5-chloro-4-yl)-2-trityloxyimino-acetylamino]-8-oxo-5-thia-1-aza-bi cyclo[4,2,0]oct-2-ene-2-carboxylic acid benzhydryl ester was dissolved in 10mℓ of DMF and the solution was cooled down to 0°C. To this solution was added 1.5mℓ of aliquots of solution prepared by dissolving 83mℓ of trimethyl amine and 50mℓ of morpholine in 467mℓ of benzene and this mixture (A) was stirred for 30 minutes.

1g of 4-chloromethylsulfanyl-6-methyl-pyrimidine-2-amine was dissolved in 10mℓ of acetone and 2g of sodium iodide was added thereto. The resulting mixture was stirred for 20 minutes at 40∼50°C and diluted with ethyl acetate and washed with water. Then, Magnesium sulfate was added thereto. The mixture was filtered and the solvent was removed under reduced pressure to obtain 4-iodomethylsulfanyl-6-methyl-pyrimidine-2-amine.

The obtained 4-iodomethylsulfanyl-6-methyl-pyrimidine-2-amine was added to mixture (A) and the whole mixture was stirred for 30 minutes. Then, this mixture was diluted with ethyl acetate and washed with saline water. The solvent was removed under reduced pressure. Solid obtained by addition of ethyl acetate to the residue followed by filtration was dissolved in 2mℓ of anisole. 4mℓ of trifluoroacetic acid was added thereto and the whole mixture was stirred for 2 hours. This mixture was cooled down to 0°C again and solid obtained by addition of 40mℓ of diethyl ether to the mixture followed by filtration was purified by preparative high pressure liquid chromatography to obtain 104mg(yield: 12%) of the title compound.
¹H NMR(D₂O) δ 2.26(s, 3H), 3.60(d, 1H, 17.0Hz), 3.82(d, 1H, 17Hz), 4.49(q, 2H, 13.7Hz), 5.18(d, 1H, 4.6Hz), 5.85(d, 1H, 5.0Hz), 6.64(s, 1H)
Mass(FAB, m/e): 588

### Example 10: Synthesis of 7-[(Z)-2-(2-amino-thiazol-5-chloro-4-yl)-2-hyd roxyimino-acetylamino]-3-(4-amino-pyrimidin-2-ylsulfanylmethylsulfanyl) -8-oxo-5-thia-1-aza-bicyclo[4,2,0]oct-2-ene-2-carboxylic acid

74mg of the title compound was obtained using 2-chloromethylsulfanyl-pyrimidine-4-amine according to the same procedure as example 9.
¹H NMR(D₂O)δ 3.65(d, 1H, 17.8Hz), 3.87(d, 1H, 17.5Hz), 4.38(d, 1H, 14.2Hz), 4.64(d, 1H, 13.8Hz), 5.15(d, 1H, 4.6Hz), 5.82(d, 1H, 5.0Hz), 6.37(d, 1H, 6.4Hz), 7.95(d, 1H, 6.4Hz)
Mass(FAB, m/e): 574

### Example 11: Synthesis of 7-[(Z)-2-(2-amino-thiazol-5-chloro-4-yl)-2-hyd roxyimino-acetylamino]-3-(2-amino-6-hydroxy-pyrimidin-4-ylsulfanylmet hylsufanyl)-8-oxo-5-thia-1-aza-bicyclo[4,2,0]oct-2-ene-2-carboxylic acid

800mg(0.82 mmol) of 7-[(Z)-2-(2-t-butoxycarbonylamino-5-chlorothiazol-4-yl)-2-trityloxyimino-acetylamino]-8-oxo-3-( 1-chloromethylthio)-5-thi a-1-aza-bicyclo[4,2,0]oct-2-ene-2-carboxylic acid benzhydryl ester prepared in preparation 10 was dissolved in 10mℓ of acetone. 246mℓ of sodium iodide was added to this solution and the mixture was stirred for 1 hour at room temperature. The solvent was removed under reduced pressure. The residue was dissolved in 10mℓ of DMF and then, 235mg of 2-amino-4-mercapto-6-hydroxypyrimidine was added thereto and the whole mixture was stirred overnight. Then, this mixture was diluted with ethyl acetate and washed with saline water. The solvent was removed under reduced pressure. Solid obtained by addition of ethyl ester to the residue followed by filtration was dissolved in 1 mℓ of anisole. To this solution was added 2mℓ of trifluoroacetic acid and the mixture was stirred for 2 hours and cooled down to 0°C again. Solid obtained by addition of 40mℓ of diethylether to the mixture followed by filtration was purified by preparative high pressure liquid chromatography to obtain 65mg(yield: 14%) of the title compound.
¹H NMR(D₂O)δ 3.63(d, 1H, 17.4Hz), 3.85(d, 1H, 17.4Hz), 4.34(d, 1H, 13.8Hz), 4.45(d, 1H, 13.7Hz), 5.21(d, 1H, 4.6Hz), 5.85(d, 1H, 6.2Hz)
Mass(FAB, m/e): 590

### Example 12: Synthesis of 7-[(Z)-2-(2-amino-thiazol-5-chloro-4-yl)-2-hyd roxyimino-acetylamino]-3-(4,6-diamino-pyrimidin-2-ylsulfanylmethylsulfa nyl)-8-oxo-5-thia-1-aza-bicycle[4,2,0]act-2-ene-2-carboxylic acid

75mg of the title compound was obtained using 4,6-diamino-2-mercaptopyrimidine according to the same procedure as example 11.
¹H NMR(D₂O)δ 3.65(d, 1H, 17.2Hz), 3.84(d, 1H, 17.4Hz), 4.48(d, 1H, 12Hz), 4.58(d, 1H, 12.5Hz), 5.18(d, 1H, 5.0Hz), 5.52(s, 1H), 5.83(d, 1H, 5.2Hz)
Mass(FAB, m/e): 589

### Example 13: Synthesis of 7-[(Z)-2-(2-amino-thiazol-5-chloro-4-yl)-2-hyd roxyimino-acetylamino]-3-(4-amino-6-hydroxy-pyrimidin-2-ylsulfanylmet hylsulfanyl)-8-oxo-5-thia-1-aza-bicycle[4,2,0]oct-2-ene-2-carboxylic acid

80mg of the title compound was obtained using 4-amino-2-mercapto-6-hydroxypyrimidine according to the same procedure as example 11.
¹H NMR(D₂O) δ 3.68(d, 1H, 17.4Hz), 3.99(d, 1H, 17.2Hz), 4.62(s, 2H), 5.22(d, 1H, 4.6Hz), 5.25(s, 1H), 5.88(d, 1H, 4.2Hz)
Mass(FAB, m/e): 590

### Example 14: Synthesis of 7-[(Z)-2-(2-amino-thiazol-5-chloro-4-yl)-2-hyd roxyimino-acetylamino]-3-(4,5-diamino-2-hydroxy-pyrimidin-6-ylsulfanyl methylsulfanyl)-8-oxo-5-thia-1-aza-bicyclo[4,2,0]oct-2-ene-2-carboxylic acid

21mg of the title compound was obtained using 4,5-diamino-6-mercapto-2-hydroxypyrimidine according to the same procedure as example 11.
¹H NMR(D₂O)δ 3.51(d, 1H, 17.4Hz), 3.77(d, 1H, 17.3Hz), 4.39(q, 2H, 11Hz), 5.20(d, 1H, 5.0Hz), 5.88(d, 1H, 5.2Hz)
Mass(FAB, m/e): 605

### Example 15: Synthesis of 7-[(Z)-2-(2-amino-thiazol-5-chloro-4-yl)-2-hyd roxyimino-acetylamino]-3-(2,5-diamino-6-hydroxy-pyrimidin-4-ylsulfanyl methylsulfanyl)-8-oxo-5-thia-1-aza-bicyclo[4,2,0]oct-2-ene-2-carboxylic acid

41mg of the title compound was obtained using 2,5-diamino-6-mercapto-2-hydroxy-pyrimidine according to the same procedure as example 11.
¹H NMR(D₂O) δ 3.46(d, 1H, 17.8Hz), 3.75(d, 1H, 17.9Hz), 4.45(d, 2H, 14.2Hz), 4.52(d, 1H, 14.2Hz), 5.18(d, 1H, 5.0Hz), 5.86(d, 1H, 4.6Hz)
Mass(FAB, m/e): 605

### Example 16: Synthesis of 7-{[2-(2-amino-5-chloro-1,3-thiazol-4-yl)-2-(me thoxyimino)acetyl]amino}-3-({[(2,6-diamino-4-pyrimidinyl)sulfanyl]methyl }sulfanyl)-8-oxo-5-thia-1-aza-bicyclo[4,2,0]oct-2-ene-2-carboxylic acid (E) -2-(ethoxycarbonyl)-2-pentenyl ester

110mg(0.1821 mmol) of 7-{[2-(2-amino-5-chloro-1,3-thiazol-4-yl)-2-(methoxyimino)acetyl]amino}-3-({[(2,6-diamino-4-pyrimidinyl)sulfanyl]methy l}sulfanyl)-8-oxo-5-thia-1-aza-bicyclo[4,2,0]oct-2-ene-2-carboxylic acid was dissolved in 3mℓ of DMF. 127mg(0.5776 mmol) of ethyl (z)-2-(bromomethyl)-2-pentenoate was slowly added dropwise to this solution at 5°C. The reaction was performed at 5°C∼10°C while stirring for two and half hours. Then, excess ethyl acetate was added thereto. The organic layer was washed with water and saline water and dried over magnesium sulfate. Then, the organic layer was filtered and distilled under reduced pressure to have 10mℓ of residual solvent. The residue was stirred at room temperature and 3∼4 drops of hydrochloric acid/ethanol solution were added dropwise thereto. The mixture was stirred for 30 minutes and filtered to obtain solid. The solid was washed with ethyl acetate three times and dried under nitrogen gas to obtain the title compound(yield: 57.9%).
¹H NMR(DMSO)δ 9.60∼9.58(d, 1H), 7.39(br, s, 1H), 6.97∼ 6.95(m, 1H)_{,} 5.98(br, s, 1H), 5.77∼5.75(m, 1H), 5.18∼5.17(d, 1H, J=5.05Hz), 4.9(q, 2H), 4.6(m, 2H), 4.13(q, 2H), 3.86(s, 3H), 1.21∼ 1.17(m, 5H), 1.0(m, 3H)
Mass(m/e): 744

### Example 17: Synthesis of 7-{[2-(2-amino-5-chloro-1,3-thiazol-4-yl)-2-(me thoxyimino)acetyl]amino}-3-({[(2,6-diamino-4-pyrimidinyl)sulfanyl]methyl }sulfanyl)-8-oxo-5-thia-1-aza-bicyclo[4,2,0]oct-2-ene-2-carboxylic acid [(2, 2-dimethylpropanoyl)oxy]methyl ester

110mg(0.1821 mmol) of 7-{[2-(2-amino-5-chloro-1,3-thiazol-4-yl)-2-(methoxyimino)acetyl]amino}-3-({[(2,6-diamino-4-pyrimidinyl)sulfanyl]methy 1}sulfanyl)-8-oxo-5-thia-1-aza-bicyclo[4,2,0]oct-2-ene-2-carboxylic acid was dissolved in 2mℓ of DMF and the solution was cooled down to -20°C. 58mg(0.2367 mmol) of pivaloyliodide dissolved in 0.8mℓ of DMF was slowly added dropwise to this solution. The reaction was performed at -20°C∼-15°C while stirring for 1 hour. Then, excess ethyl acetate was added thereto. The organic layer was washed with water and saline water and dried over magnesium sulfate. Then, the organic layer was filtered and distilled under reduced pressure to have 10mℓ of the residual solvent. The residue was stirred at room temperature and 3∼4 drops of hydrochloric acid/ethanol solution were added dropwise thereto at room temperature. Then, the mixture was stirred for 30 minutes again and filtered to obtain solid. The solid was washed with ethyl acetate three times and dried under nitrogen gas to obtain the title compound(yield: 59.7%).
¹H NMR(DMSO)δ 9.62∼9.60(d, 1H), 6.03(s, H), 5.83∼5.75(m, 3H), 5.22∼5.21(d, 1H, J=5.04Hz), 3.9(m, 2H), 3.86(s, 3H), 1.14(s 9H)
Mass(m/e): 718

### Example 18: Synthesis of 7-{[2-(2-{[(2S)-2-amino-2-phenylacetyl]amino} -5-chloro-1,3-thiazol-4-yl)-2-(methoxyimino)acetyl]amino}-3-({[(2,6-diamin o-4-pyrimidinyl)sulfanyl]methyl}sulfanyl)-8-oxo-5-thia-1-aza-bicyclo[4,2,0] oct-2-ene-2-carboxylic acid

600mg (0.673 mmol) of 3-(acetylsulfanyl)-7-{[2-[2-({[(2S)-2-[(t-but -oxycarbonyl)amino]-2-phenylacetyl}amino)-5-chloro-1,3-thiazol-4-yl]-2-(met hoxyimino)acetyl]amino}-8-oxo-5-thia-1-aza-bicyclo[4,2,0]oct-2-ene-2-carbox ylic acid benzhydryl ester was dissolved in 6mℓ of DMF and the solution was cooled down to 0°C. Then, 2mℓ of aliquots of the solution prepared by dissolving 0.467mℓ of triethylamine and 0.293mℓ of morpholine in 10 mℓ of benzene was added thereto and this mixture (A) was stirred for 30 minutes.

204mg(1.2 equivalents) of 2-chloromethylsulfanyl pyrimidine was dissolved in 2mℓ of acetone and to this solution was added 400mg of sodium iodide. The mixture was stirred for 3 hours at 40∼50°C and diluted with ethyl acetate and washed with water. Magnesium sulfate was added thereto. The resulting mixture was filtered and the solvent was removed under reduced pressure to obtain 2-iodomethylsulfanyl pyrimidine.

The obtained 2-iodomethylsulfanyl pyrimidine was added to mixture (A) and the whole mixture was stirred for 30 minutes and then, diluted with ethyl acetate and washed with saline water. The solvent was removed under reduced pressure. Solid obtained by addition of ethyl ester to the residue followed by filtration was dissolved in 1.5mℓ of anisole. To this solution was added 1mℓ of trifluoroacetic acid. The mixture was stirred for 60 minutes and cooled down to 0°C. Solid obtained by addition of 10mℓ of diethyl ether thereto followed by filtration was purified by preparative high pressure liquid chromatography to obtain 100mg(yield: 21%) of the title compound.
¹H NMR(D₂O) δ 7.45∼7.42(br, m, 5H), 6.02(m, 1H), 5.88∼ 5.86(m, 1H), 5.18∼5.16(m, III), 4.40∼4.35(m, 2H), 4.05(s, 3H), 3.82(m, 1H), 3.66(m, 1H), 3.57(m, 1H)
Mass( m/e): 750

### Example 19: Synthesis of 7-[(Z)-2-(2-amino-1,3-thiazol-4-yl)-2-methoxyi mino-acetylamino]-3-(2,4-diaminopyrimidin-5-ylmethylsulfanyl)-8-oxo-5-t hia-1-aza-bicyclo[4,2,0]oct-2-ene-2-carboxylic acid

1.0g(1.12 mmol) of 3-acetylsulfanyl-7-{2-methoxyimino-2-[2-(trityl -amino)-1,3-thiazol-4-yl]acetylamino}-8-oxo-5-thia-1-aza-bicyclo[4,2,0]oct-2-e ne-2-carboxylic acid benzhydryl ester was dissolved in 9mℓ of DMF and the solution was cooled down to 0 °C. To this solution was added 23.3 mℓ of aliquots of solution prepared by dissolving 0.467mℓ of triethyl amine and 0.293 mℓ of morpholine in 10mℓ of benzene and the whole mixture was stirred for 30 minutes. 490mg(1.2 equivalents) of 2,4-diamino-5-bromomethyl pyrimidine hydrobromide was added thereto and then, triethyl amine(1.0 equivalent) was slowly added dropwise thereto. The resulting mixture was stirred for 30 minutes at 0°C and diluted with ethyl acetate and washed with water. This mixture was dried over magnesium sulfate and the solvent was removed under reduced pressure. Solid obtained by addition of ethyl ester to the residue followed by filtration was dissolved in 1.5mℓ of anisole. To this solution was added 3mℓ of trifluoroacetic acid and the whole mixture was stirred for 60 minutes and then, cooled down to 0°C. The solid obtained by addition of 20mℓ of diethyl ether thereto followed by filtration was purified by preparative high pressure liquid chromatography to obtain 150mg(yield: 25%) of the title compound.
¹H NMR(D₂O) δ 3.37(d, 1H, 18Hz), 3.64(d, 1H, 18Hz), 3.55(d, 1H, 14Hz), 3.89(d, 1H, 14Hz), 4.02(s, 3H), 5.17(d, 1H, 4.5Hz), 5.80(d, 1H, 4.5Hz), 7.03(s, 1H), 7.0(s, 1H)
Mass(FAB, m/e) : 538

### Example 20: Synthesis of 7-[(Z)-2-(2-amino-1,3-thiazol-4-yl)-2-methoxyi mino-acetylamino]-3-(2,4-diaminopyrimidin-6-ylmethylsulfanyl)-8-oxo-5-t hia-1-aza-bicyclo[4,2,0]oct-2-ene-2-carboxylic acid

623mg(0.70 mmol) of 3-acetylsulfanyl-7-{2-methoxyimino-2-[2-(trityl -amino)-1,3-thiazol-4-yl]acetylamino}-8-oxo-5-thia-1-aza-bicyclo[4,2,0]oct-2-e ne-2-carboxylic acid benzhydryl ester was dissolved in 6mℓ of DMF and the solution was cooled down to 0°C. Then, 2.1mℓ of aliquots of solution prepared by dissolving 0.467mℓ of triethyl amine and 0.293mℓ of morpholine in 10mℓ of benzene was added thereto and the whole mixture was stirred for 30 minutes. 281mg(1.1 equivalents) of 2,4-diamino-6-bromomethyl pyrimidne hydrobromide was added thereto and then, triethyl amine(1.0 equivalent) was slowly added dropwise thereto. The resulting mixture was stirred for 30 minutes at 0°C and then, diluted with ethyl acetate and washed with water. The mixture was dried over magnesium sulfate and the solvent was removed under reduced pressure. Solid obtained by addition of ethyl ester to the residue followed by filtration was dissolved in 1.5mℓ of anisole. To this solution was added 1mℓ of trifluoroacetic acid. This mixture was stirred for 60 minutes and cooled down to 0°C. Solid obtained by addition of 20 mℓ of diethyl ether thereto followed by filtration was purified by preparative high pressure liquid chromatography to obtain 150mg(yield: 40%) of the title compound.
¹H NMR(D₂O) δ 3.35(d, 1H, 14.5Hz), 3.60(d, 1H, 14.5Hz), 3.70(d, 1H, 14.5Hz), 3.73(d, 1H, 14.5Hz), 4.00(s, 3H), 5.17(d, 1H, 4.5Hz), 5.80(d, 1H, 4.5Hz), 7.03(s, 1H), 6.71(s, 1H)
Mass(FAB, m/e) : 538

### Example 21: Synthesis of 7-[(Z)-2-(2-amino-5-chloro-1,3-thiazol-4-yl)-2-methoxyimino-acetylamino]-3-(2,4-diaminopyrimidin-5-ylmethylsulfanyl)-8-oxo-5-thia-1-aza-bicyclo[4,2,0]oct-2-ene-2-carboxylic acid

820mg(0.911 mmol) of 3-(acetylsulfanyl)-7-{2-(5-chloro-[2-(tritylami -no)-1,3-thiazol-4-yl]-2-(methoxyimino)acetyl)amino}-8-oxo-5-thia-1-aza-bicy clo[4,2,0]oct-2-ene-2-carboxylic acid benzhydryl ester was dissolved in 9 mℓ of DMF and the solution was cooled down to 0°C. 1.0mℓ of aliquots of solution prepared by dissolving 0.467mℓ of triethylamine and 0.293mℓ of morpholine in 10mℓ of benzene and the mixture was stirred for 30 minutes. 400mg(1.2 equivalent) of 2,4-diamino-5-bromomethyl pyrimidine hydrobromide was added thereto and then, triethylamine(1.0 equivalent) was slowly added dropwise thereto. This mixture was stirred for 30 minutes at 0°C and then, diluted with ethyl acetate and washed with water. The mixture was dried over magnesium sulfate and the solvent was removed under reduced pressure. Solid obtained by addition of ethyl ester to the residue followed by filtration was dissolved in 1.5mℓ of anisole and 3mℓ of trifluoroacetic acid was added thereto. The resulting mixture was stirred for 60 minutes and cooled down to 0°C. Solid obtained by addition of 20mℓ of diethyl ether thereto followed by filtration was purified by preparative high pressure liquid chromatography to obtain 150mg(yield: 25%) of the title compound.
¹H NMR(D₂O) δ 3.35(d, 1H, 18Hz), 3.64(d, 1H, 18Hz), 3.55(d, 1H, 14Hz), 3.90(d, 1H, 14Hz), 4.02(s, 3H), 5.17(d, 1H, 4.5Hz), 5.80(d, 1H, 4.5Hz), 7.70(s, 1H).
Mass(FAB, m/e) : 572

### Example 22: Synthesis of 7-[(Z)-2-(2-amino-5-chloro-1,3-thiazol-4-yl)-2-hydroxyimino-acetylamino]-3-(2,4-diaminopyrimidin-6-ylmethylsulfanyl) 8-oxo-5-thia-1-aza-bicyclo[4,2,0]oct-2-ene-2-carboxylic acid

1.0g(1.063 mmol) of 3-(acetylsulfanyl)-7-({{2-{2-[(t-butoxycarbonyl) -amino]-5-chloro-1,3-thiazol-4-yl}-2-(trityloxy)imino}acetyl}amino)-8-oxo-5-t hia-1-aza-bicyclo[4,2,0]oct-2-ene-2-carboxylic acid benzhydryl ester was dissolved in 10mℓ of DMF and the solution was cooled down to 0°C. To this solution was added 1.1mℓ of aliquots of solution prepared by dissolving 0.467mℓ of triethylamine and 0.293 mℓ of morpholine in 10mℓ of benzene and the mixture was stirred for 30 minutes. 467mg(1.2 equivalents) of 2,4-diamino-6-bromomethyl pyrimidine hydrobromide was added thereto. This mixture was stirred for 30 minutes at 0°C and then, diluted with ethyl acetate and washed with water. The mixture was dried over magnesium sulfate and the solvent was removed under reduced pressure. Solid obtained by addition of ethyl ester to the residue followed by filtration was dissolved in 1.5mℓ of anisole and 1mℓ of trifluoroacetic acid was added thereto. The mixture was stirred for 60 minutes and cooled down to 0°C again. Solid obtained by addition of 20mℓ of diethyl ether thereto followed by filtration was purified by preparative high pressure liquid chromatography to obtain 150mg(yield: 40%) of the title compound.
¹H NMR(D₂O) δ 3.35(d, 1H, 14.5Hz), 3.60(d, 1H, 14.5Hz), 3.70(d, 1H, 14.5Hz), 3.73(d, 1H, 14.5Hz), 5.15(d, 1H, 4.5Hz), 5.82(d, 1H, 4.5Hz), 7.71(s, 1H).
Mass(FAB, m/e) : 558

### Example 23: Synthesis of 7-[(Z)-2-(2-amino-1,3-thiazol-4-yl)-2-methoxyi mino-acetylamino]-3-(2,4-diaminopyrimidin-5-ylmethylsulfanyl)-8-oxo-5-t hia-1-aza-bicyclo[4,2,0]oct-2-ene-2-carboxylic acid

To 10mℓ of THF:DMF solvent(4:1) was added 503mg(1.5 mmol) of 2,4-diamino-5-bromomethyl dihydrobromide and the mixture was cooled down to 0 °C. Lithium thioacetate(3 equivalents) dissolved in methanol was slowly added dropwise thereto. Completion of the reaction was confirmed by TLC. Then, NaOMe(1.0 equivalent) dissolved in methanol was added dropwise thereto at 0°C. After 10 minutes, the mixture was cooled down to -78°C and 927mg(0.7 equivalents) of 4-methoxybenzyl-3 -(iodomethyl)-7-( {2-(methoxyimino)-2-[2-(tritylamino)-1,3 -thiazol-4-yl]acetyl}amino)-8-oxo-5-thia-1-aza-bicyclo[4,2,0]oct-2-ene-2-carbo xylate dissolved in THF was slowly added dropwise thereto. The reaction was performed for 30 minutes and stopped with 1N of hydrochloric acid solution. The reaction mixture was diluted with ethyl acetate and washed with saline water. The organic layer was dried over magnesium sulfate and filtered. Then, the organic layer was distilled under reduced pressure and triturated with dichloromethane and diethyl ether to obtain a compound. The obtained compound was dissolved in 1 mℓ of anisole and the solution was cooled down to 0°C. Then, trifluoroacetic acid was slowly added dropwise thereto and then, the mixture was warmed up to room temperature again. Completion of the reaction was confirmed by HPLC and the reaction mixture was cooled down to 0°C. 20mℓ of diethyl ether was added thereto and the mixture was filtered to obtain solid. The solid was purified by preparative high pressure liquid chromatography to obtain 20 mg of the title compound.
¹H NMR(D₂O) δ 3.45(m, 1H), 3.58-3.65(m, 5H), 4.05(s, 3H), 5.15(m, 1H), 7.06(s, 1H), 7.79-7.78(d, 1H, J=4.6), 7.73(s, 1H).
Mass(FAB, m/e) : 552

### Example 24: Synthesis of 7-[(Z)-2-(2-amino-5-chloro-1,3-thiazol-4-yl)-2-hydroxyimino-acetylamino]-3-(2,4-diaminopyrimidin-5-ylmethylsulfanylm ethyl)-8-oxo-5-thia-1-aza-bicyclo[4,2,0]oct-2-ene-2-carboxylic acid

To 10mℓ of THF:DMF(4:1) solvent was added 839mg(2.5 mmol) of 2,4-diamino-5-bromomethyl dihydrobromide and the mixture was cooled down to 0°C. Lithium thioacetate(3 equivalents) dissolved in methanol was slowly added dropwise thereto. Completion of the reaction was confirmed by TLC and then, NaOMe(1.0 equivalent) dissolved in methanol was added dropwise thereto at 0°C. After 10 minutes, the reaction mixture was cooled down to -78 °C and 1.94g(0.8 equivalents) of 4-methoxybenzyl-7-({2-{2-[(t-butoxycarbonyl)amino]-5-chloro-1,3-thiazol -4-yl-2-[(trityloxy)imino]acetyl}amino)-3-(iodomethyl)-8-oxo-5-thia- 1-aza-bic yclo[4,2,0]oct-2-ene-2-carboxylate dissolved in THF was slowly added dropwise thereto. The reaction was performed for 30 minutes and stopped with IN of hydrochloric acid solution. The reaction mixture was diluted with ethyl acetate and washed with saline water. The organic layer was dried over magnesium sulfate and filtered. The organic layer was distilled under reduced pressure and triturated by dichloromethane and diethyl ether to obtain a compound. The obtained compound was dissolved in 1mℓ of anisole and the solution was cooled down to 0°C. Then, fluoroacetic acid was slowly added dropwise thereto and the mixture was warmed up to room temperature. Completion of the reaction was confirmed by HPLC and the reaction mixture was cooled down to 0°C. 20mℓ of diethyl ether was added thereto and the mixture was filtered to obtain solid. The solid was purified by preparative high pressure liquid chromatography to obtain 30mg of the title compound.
¹H NMR(D₂O) δ 3.31(m, 1H), 3.58-3.54(m, 5H), 5.15-5.16(d, 1H, J=4.6), 5.81(d, 1H, J=4.55), 7.65(s, 1H).
Mass(FAB, m/e) : 572

### Example 25: Synthesis of 7-[(Z)-2-(2-amino-thiazol-4-yl)-2-methoxyimin o-acetylamino]-3-(2,6-diamino-pyrimidin-4-ylsulfanyl)-8-oxo-5-thia-1-azabicyclo[4,2,0]oct-2-ene-2-carboxylic acid

75mg(1.0 equivalent) of diethylthio phosphoryl (Z)-(2-aminothiazol-4-yl)methoxyimino acetic acid and 100mg(0.22 mmol) of 7-amino-3-(2,6-diamino-pyrimidin-4-ylsulfanyl)-8-oxo-5-thia-1-aza-bicyclo [4,2,0]oct-2-ene-carboxylic acid were dissolved in 1mℓ of acetone and 1mℓ of water. The solution was cooled down to 0°C and 132µℓ of tetrabutyl amine was added thereto. The mixture was warmed up to 15°C and stirred for 5 hours. The solvent was removed under reduced pressure. The residue was purified by preparative high pressure liquid chromatography to obtain 70mg(yield: 60%) of the title compound.
¹H NMR(D₂O) δ 3.48(d, 1H, 7.6Hz), 3.89(d, 1H, 7.6Hz), 4.08(s, 3H), 5.41(d, 1H, 4.9Hz), 5.81(d, 1H, 4.9Hz), 5.85(s, 1H), 7.02(s, 1H)
Mass(FAB, m/e): 523

### Example 26: Synthesis of 7-[(Z)-2-(2-amino-thiazol-4-yl)-2-hydroxyimin o-acetylamino]-3-(2,6-diamino-pyrimidin-4-ylsulfanyl)-8-oxo-5-thia-1-azabicyclo[4,2,0]oct-2-ene-2-carboxylic acid

300mg(0.66 mmol) of 7-amino-3-(2,6-diamino-pyrimidin-4-ylsulfany -1)-8-oxo-5-thia-1-aza-bicyclo[4,2,0]oct-2-ene-2-carboxylic acid was dissolved in 1mℓ of methylene chloride and the solution was cooled down to 0°C under nitrogen gas. Then, 654µℓ(4.0 equivalents) of N,O-bistrimethylsilylacetamide was added thereto and the mixture was stirred at room temperature to be completely dissolved. The solution was cooled down to 0°C again and 534mg of diethyithiophosphoryl (Z)-(2-aminothiazol-4-yl-triphenyloxyimino acetic acid and 16µℓ of pyridine were added thereto and the whole mixture was stirred for 3 hours at 15°C. 20mℓ of diethyl ether and 4mℓ of water were added thereto and the resulting mixture was stirred for 30 minutes and filtered to obtain solid. The obtained solid was dissolved in 0.5mℓ of anisole and 1mℓ of trifluoroacetic acid was added thereto. The mixture was stirred for 30 minutes and cooled down to 0°C again. 10mℓ of diethyl ether was added thereto and the mixture was filtered to obtain solid. The solid was purified by preparative high pressure liquid chromatography to obtain 70mg(yield: 21%) of the title compound.
¹H NMR(D₂O) δ 3.47(d, 1H, 7.6Hz), 3.91(d, 1H, 7.6Hz), 5.42(d, 1H, 4.9Hz), 5.80(s, 1H), 5.89(d, 1H, 4.9Hz), 7.00(s, 1H)
Mass(FAB, m/e): 509

### Example 27: Synthesis of 7-[(Z)-2-(2-amino-thiazol-4-yl)-2-ethoxyiminoacetylamino]-3-(2,6-diamino-pyrimidin-4-ylsulfanyl)-8-oxo-5-thia-1-aza-bi cyclo[4,2,0]oct-2-ene-2-carboxylic acid

85mg of the title compound was obtained using diethylthio phosphoryl (Z)-(2-aminothiazol-4-yl)-ethoxyimino acetic acid according to the same procedure as example 26.
¹H NMR(D₂O) δ 1.30(t, 3H, 7.3Hz), 3.52(d, 1H, 7.6Hz), 3.88(d, 1H, 7.6Hz), 4.30(q, 2H, 6.3Hz), 5.40(d, 1H, 4.9Hz), 5.80(s, 1H), 5.88(d, 1H, 4.9Hz), 7.06(s, 1H)
Mass(FAB, m/e): 537

### Example 28: Synthesis of 7-[(Z)-2-(2-amino-thiazol-4-yl)-2-propyn-2-yio xyimino-acetylamino]-3-(2,6-diamino-pyrimidin-4-ylsulfanyl)-8-oxo-5-thia1-aza-bicyclo[4,2,0]oct-2-ene-2-carboxylic acid

80mg of the title compound was obtained using diethylthiophosphoryl (Z)-(2-aminothiazol-4-yl-propyn-2-yloxyimino acetic acid according to the same procedure as example 26.
¹H NMR(D₂O) δ 3.0(s, 1H), 3.52(d, 1H, 7.6Hz), 3.88(d, 1H, 7.6Hz), 4.91(s, 2H), 5.38(d, 1H, 4.9Hz), 5.80(s, 1H), 5.89(d, 1H, 4.9Hz), 7.01(s, 1H)
Mass(FAB, m/e): 547

### Example 29: Synthesis of 8-[(Z)-2-(5-amino-[1,2,4]-thiadiazol-3-yl)-2-(et hoxyimino-acetylamino]-4-(2,6-diamino-pyrimidin-4-ylsulfanyl)-7-oxo-2-th ia-bicyclo[4,2,0]oct-4-ene-5-carboxylic acid

75mg of the title compound was obtained using diethylthiophosphoryl (Z)-(5-amino-[1,2,4]-thiadiazol-3-yl)-ethoxyimino acet -ic acid according to the same procedure as example 26.
¹H NMR(D₂O) δ 1.40(t, 3H, 7.2Hz), 3.52(d, 1H, 7.5Hz), 3.91(d, 1H, 7.5Hz), 4.42(q, 2H, 6.2Hz), 5.41(d, 1H, 4.8Hz), 5.80(s, 1H), 5.92(d, 1H, 4.8Hz)
Mass(FAB, m/e): 538

### Example 30: Synthesis of 7-[(z)-2-(2-amino-thiazol-4-yl)-2-hydroxyimino -acetylamino]-3-(2-amino-6-hydroxy-pyrimidin-4-ylsulfanyl)-8-oxo-5-thia-1-aza-bicyclo[4,2,0]oct-2-ene-2-carboxylic acid

800mg(1.087 mmol) of benzhydryl 7-amino-3-[(2-amino-6-hydroxy -4-pyrimidinyl)sulfanyl]-8-oxo-5-thia-1-aza-bicyclo[4,2,0]oct-2-ene-2-carboxyl ate was stirred for 1 hour in anisole/trifluoroacetic acid(1/2). Excess ether was added thereto and the mixture was filtered and dried to obtain solid compound. The obtained solid compound was used in the subsequent reaction without further purification.

To the obtained compound was added 2mℓ of dichloromethane and the mixture was cooled down to 0°C. 550µℓ(4.348 mmol) of N,O-bistrimethylsilyl acetamide was added thereto and the mixture was stirred for 10 minutes at 0°C to be completely dissolved. 741mg(1.087 mmol) of O,O-diethyl 2-[2-(tritylamino)-1,3-thiazol-4-yl]-2-[(trityloxy)imino]acetylphosphonothioate dissolved in 3mℓ of dichloromethane was slowly added dropwise thereto and 39µℓ(0.542 mmol) of pyridine was further added thereto. The reaction was performed at 5∼10°C while stirring for 3 hours. 5 or 6 drops of water was added to the reaction mixture and the mixture was stirred for 5 minutes. Excess ether was added thereto and the resulting mixture was filtered to obtain solid. Solid was washed with ether several times and dried. Then the solid was deprotected with anisole/trifluoroacetic acid(1/2) and isolated and purified by preparative high pressure liquid chromatography to obtain the title compound(yield of 3 steps: 13.5%).
¹H NMR(D₂O) δ 6.99(s, 1H), 5.91(d, 1H, J=4.9Hz), 5.61(s, 1H), 5.38(d, 1H, J=5.0Hz), 3.89(d, 1H, J=17.9Hz), 3.48(d, 1H, J=17.9Hz)
Mass(m/e) 510

### Example 31: Synthesis of 7-[(z)-2-(2-amino-thiazole-5-chloro-4-yl)-2-hyd roxyimino]-3-(2-amino-6-hydroxy-pyrimidin-4-ylsulfanyl)-8-oxo-5-thia-1-a za-bicyclo[4,2,0]oct-2-ene-2-carboxylic acid

The title compound(yield of the 3 steps: 15.1%) was obtained using 800mg(1.087 mmol) of benzhydryl 7-amino-3-[(2-amino-6-hydroxy-4 -pyrimidinyl)sulfanyl]-8-oxo-5-thia-1-aza-bicyclo[4,2,0]oct-2-ene-2-carboxylat e and 780mg(1.087 mmol) of O,O-diethyl 2-[5-chloro-2-(tritylamino)-1,3-thiazol-4-yl]-2-[(trityloxy)imino]acetylphosphonothioate according to the same procedure as example 30.
¹H NMR(D₂O) δ 5.98-5.97(d, 1H, J=5.05Hz), 5.70(s, 1H), 5.42-5.41(d, 1H, J-5.05Hz), 3.95-3.91(ABq, 1H, J=17.5Hz), 3.57-3.53(ABq, 1H, J=17.6Hz)
Mass(m.e) 544

### Example 32: Synthesis of 7-[(z)-2-(2-amino-thiazole-5-chloro-4-yl)-2-hyd roxyimino-acetylamino]-3-(2,6-diaminopyrimidin-4-ylsulfanyl)-8-oxo-5-thi a-1-aza-bicyclo[4,2,0]oct-2-ene-2-carboxylic acid

The title compound(yield of 3 steps: 13.5%) was obtained using 800mg(1.087 mmol) of benzhydryl 7-amino-3-[(2,6-diamino-4-pyrimidinyl) sulfanyl]-8-oxo-5-thia-1-aza-bicyclo[4,2,0]oct-2-ene-2-carboxylate and 780mg(1.087 mmol) of O,O-diethyl 2-[5-chloro-2-(tritylamino)-1,3-thiazol-4-yl]-2-[(trityloxy)imino]acetylphosphonothioate according to the same procedure as example 30.
¹H NMR(D₂O) δ 5.92-5.91(d, 1H, J=5.04Hz), 5.81(s, 1H), 5.37(d, 1H, J=5.04Hz), 3.89-3.85(ABq, 1H, J=17.41Hz), 3.49-3.47(ABq, 1H, J=17.87Hz)
Mass(m/e) 543.9

### Test example 1: Measurement of Minimum Inhibitory Concentration (MIC)

Antimicrobial activity of the compounds of the present invention was evaluated by measuring Minimum Inhibitory Concentration(MIC) of Compound Nos. 1∼8, 19, 20 and 25∼29 prepared in the above examples, and known compounds, Ceftazidime and Cefdinir, as control compounds, against standard strains, respectively. Specifically, test compounds were diluted by 2-fold dilution method and dispersed in Mueller-Hinton agar medium and then, 2µℓ of test strains were inoculated at a concentration of 10⁷ cfu(colony forming unit)/mℓ on the medium and incubated for 20 hours at 37°C. The results are shown in the following tables 1a and 1b.

**Table 1a.**

| MIC against standard strains(µg/mℓ) | | | | | |
|---|---|---|---|---|---|
| | S. aureus | S. pneumoniae | E. coli | P. vulgaris | H. influenzae |
| 1 | 0.5 | 0.5 | 0.13 | 0.063 | 0.008 |
| 2 | 0.5 | 0.25 | 0.063 | 0.031 | 0.008 |
| 3 | 0.5 | 0.25 | 0.063 | 0.016 | 0.016 |
| 4 | 0.25 | 0.13 | 0.063 | 0.016 | 0.008 |
| 5 | 0.13 | 1 | 0.063 | 0.031 | 0.25 |
| 6 | 0.13 | 0.5 | 0.031 | 0.031 | 0.5 |
| 7 | 0.13 | 0.25 | 0.25 | 0.25 | 0.13 |
| 8 | 0.25 | 0.13 | 0.25 | 0.25 | 0.031 |
| 9 | 0.5 | 2 | 0.031 | 0.063 | 0.031 |
| 20 | 2 | 2 | 0.031 | 0.016 | 0.031 |
| Ceftazidime | 4 | 8 | 0.031 | 0.031 | 0.063 |
| Cefdinir | 0.031 | 4 | 0.016 | 0.031 | 0.25 |

**Table 1b.**

| MIC against standard strains(µg/mℓ) | | | | | | |
|---|---|---|---|---|---|---|
| | 25 | 26 | 27 | 28 | 29 | Ceftazi-dime |
| Staphylococcus aureus 6538p | 1 | 0.5 | 2 | 1 | 2 | 16 |
| Staphylococcus aureus giorgio | 0.5 | 0.25 | 0.5 | 0.5 | 1 | 4 |
| Staphylococcus aureus 77 | 4 | 2 | 4 | 4 | 4 | 32 |
| Staphylococcus aureus 241 | 8 | 8 | 8 | 8 | 8 | > 128 |
| Staphylococcus epidermidi 887E | 8 | 2 | 8 | 8 | 8 | > 128 |
| Enterococcus faecalis 29212 | > 64 | 16 | 64 | 32 | 64 | > 128 |
| Escherichia coli 10536 | 0.063 | 0.063 | 0.25 | 0.25 | 0.13 | 0.063 |
| Escherichia coli 3190Y | 0.031 | 0.031 | 0.13 | 0.13 | 0.063 | 0.031 |
| Escherichia coli 851E | 0.063 | 0.063 | 0.25 | 0.25 | 0.25 | 0.063 |
| Escherichia coli TEM1 1193E | 0.25 | 0.5 | 0.5 | 0.5 | 1 | 0.13 |
| Escherichia coli TEM3 3455E | 8 | 64 | 16 | 16 | 32 | 8 |
| Escherichia coli TEM9 2639E | 32 | 32 | 64 | 64 | 64 | > 128 |
| Salmonella typhimurium 14028 | 0.25 | 0.13 | 1 | 1 | 1 | 0.25 |
| Klebsiella pneumoniae 2011E | 0.5 | 1 | 2 | 2 | 2 | 0.5 |
| Klebsiella aerogenes SHV-1 1976E | 0.25 | 0.5 | 0.5 | 0.5 | 1 | 0.25 |
| Klebsiella aerogenes K1+ 1082E | > 64 | > 64 | > 64 | > 64 | > 64 | 0.25 |
| Proteus vulgaris 6059 | 0.016 | 0.031 | 0.063 | 0.031 | 0.063 | 0.031 |
| Morganella morganii 1375E | 0.13 | 0.25 | 0.25 | 0.25 | 0.25 | 0.063 |
| Citrobacter freundii 8090 | 1 | 8 | 2 | 2 | 2 | 0.13 |
| Citrobacter diversus 2046E | > 64 | > 64 | 64 | > 64 | > 64 | 0.25 |
| Enterobacter cloacae IND+VE 1194E | > 64 | > 64 | > 64 | > 64 | > 64 | 0.5 |
| Enterobacter cloacae P99 | > 64 | > 64 | > 64 | > 64 | > 64 | 32 |
| Serratia marcescens 1826E | 2 | 64 | 4 | 8 | 4 | 0.25 |
| Acinetobacter calcoaceticu 15473 | 64 | 32 | 32 | 32 | 32 | 2 |
| Moraxella catarrhalis 25240 | ≤ 0.008 | ≤ 0.008 | ≤ 0.008 | 0.016 | ≤ 0.008 | 0.016 |
| Moraxella catarrhalis C2431 | ≤ 0.008 | 0.016 | ≤ 0.008 | 0.016 | ≤ 0.008 | 0.031 |
| Pseudomonas aeruginosa 1912E | 16 | > 64 | 8 | 32 | 8 | 1 |
| Pseudomonas aeruginosa 6065Y | 64 | > 64 | 64 | 64 | > 64 | 32 |

*[not claimed in the present invention]

As shown in tables 1a and 1b, the compounds of the present invention exhibited excellent antimicrobial activity not only against Gram positive but also against Gram negative bacteria.

### Test example 2: Pharmacokinetic experiment

Male mice, each of which has a body weight of 230±10g were used in the present pharmacokinetic experiment. Test compounds were formulated into 1% of solution and then, the solution was administered by oral route and by injection with a dose of 20mg/kg, respectively. At the fixed interval of time after administration, blood was gathered from the mice. Concentrations of test compounds in blood were measured by HPLC quantitative analysis and based on the results, pharmacokinetic parameters were calculated. These are shown in the following tables 2a and 2b.

**Table 2a.**

| Pharmacokinetic parameters in mice | | | | | |
|---|---|---|---|---|---|
| | Injection | | Oral administration | | |
| | half-life (min) | AUC (µg · min/mℓ) | maximum conc.(µg/mℓ) | half-life (min) | AUC (µg • min/mℓ) |
| 7 | 139 | 6432 | 13.8 | 168 | 3593 |
| 19 | not tested | not tested | 3.2 | 69 | 379 |
| Cefdinir | 21 | 3001 | 2.9 | 74 | 383 |

**Table 2b.**

| Changes in blood concentration of Compound No. 25* with the lapse of time | | | | | | | |
|---|---|---|---|---|---|---|---|
| time (min) | Injection | | | Oral administration | | | |
| | mouse-1 | mouse-2 | average conc. (µg/mℓ) | mouse-1 | mouse-2 | mouse-3 | average conc. (µg/mℓ) |
| 1 | 120.0 | 72.8 | 96.4 | - | - | - | - |
| 5 | 51.7 | 30.7 | 41.2 | 0.07 | 0.13 | 0.06 | 0.09 |
| 10 | 37.7 | 20.4 | 29.1 | 0.16 | 0.27 | 0.14 | 0.20 |
| 20 | 21.6 | 12.7 | 17.1 | 0.28 | 0.72 | 0.27 | 0.49 |
| 40 | 12.2 | 7.3 | 9.8 | 0.27 | 0.59 | 0.35 | 0.47 |
| 60 | 7.6 | 4.6 | 6.1 | 0.16 | 0.61 | 0.26 | 0.44 |
| 90 | 4.3 | 2.4 | 3.3 | 0.07 | 0.84 | 0.29 | 0.57 |
| 120 | 2.2 | 1.3 | 1.8 | 0.06 | 0.48 | 0.24 | 0.36 |
| 180 | 1.1 | 0.6 | 0.8 | 0.06 | 0.59 | 0.24 | 0.41 |
| 240 | 0.5 | 0.3 | 0.4 | 0.06 | 0.22 | 0.23 | 0.23 |

| | | | | | | | |
|---|---|---|---|---|---|---|---|
| *[not claimed in the present invention] | | | | | | | |

As shown in tables 2a and 2b, in case of injection of test compounds, they were rapidly distributed in blood and slowly excreted. Specifically, in case of intravenous injection of test compounds, half-lifes of test compounds were longer than that of Cefdinir, i.e. 21 minutes. Also, in case of oral administration of test compounds, they exhibited high absorption rate.

Accordingly, the compounds of the present invention are cephalosporin antibiotics which exhibit potent antimicrobial activity and have broad antibacterial spectrum and remarkably improved pharmacokinetic characteristics and further, are also effective for oral administration.

This application is based on patent application Nos. 98-17260, 98-35650, 98-35651 and 99-3757 filed in the Republic of Korea, the contents of which are incorporated hereinto by references.

## Claims

1. Cephalosporin compounds of the following formula (I): 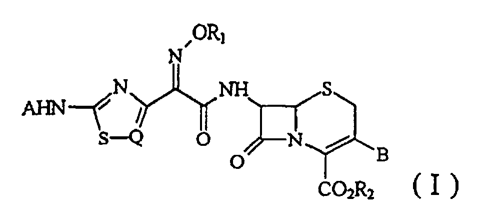 , wherein
A represents hydrogen or amino protecting group,
R₁ represents hydrogen, C₁₋₆alkyl which may be mono- to tri-substituted by C₁₋₆alkoxy, cyano, halogen, C₃₋₆cycloalkyl or C₅₋₆heteroaryl having 1 or 2 nitrogen or oxygen atom(s), C₃₋₄alkenyl, C₃₋₄alkynyl which may be substituted by amino, or C₃₋₆cycloalkyl,
R₂ represents hydrogen or carboxy protecting group,
Q represents CH, CX or N, wherein X represents halogen,
B represents a radical selected from the following group: 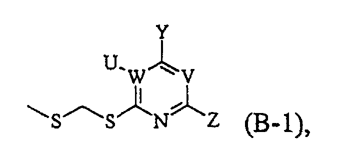 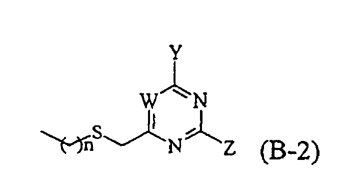 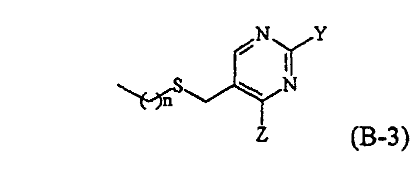 or 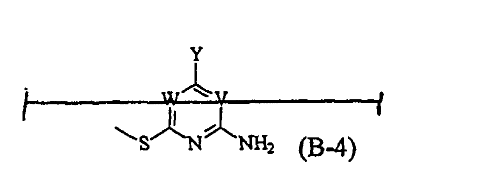 , wherein
U represents hydrogen or optionally substituted amino,
V and W independently of one another represent C or N, with the proviso that V and W are different,
Y and Z independently of one another represent hydrogen, optionally substituted amino, hydroxy or C₁₋₄alkyl, and
n is 0 or 1,
pharmaceutically acceptable nontoxic salts, physiologically hydrolyzable esters, hydrates, solvates or isomers thereof.

2. The cephalosporin compounds according to claim 1 which are selected from the group consisting of:
7-[(Z)-2-(2-amino-thiazol-4-yl)-2-methoxyimino-acetylamino]-8-oxo-3 -(pyrimidin-2-ylsulfanylmethylsulfanyl)-5-this-1-aza-bicyclo[4,2,0]oct-2-ene-2-carboxylic acid(1);
7-[(Z)-2-(2-amino-thiazol-4-yl)-2-methoxyimino-acetylamino]-8-oxo-3 -(4-amino-pyrimidin-2-ylsulfanylmethylsulfanyl)-5-thia-1-aza-bicyclo[ 4,2,0]oct-2-ene-2-carboxylic acid(2);
7-[(Z)-2-(2-amino-thiazol-4-yl)-2-methoxyimino-acetylamino]-3-(4,6-d iamino-pyrimidin-2-ylsulfanylmethylsulfanyl)-8-oxo-5-thia-1-aza-bicyc lo[4,2,0]oct-2-ene-2-carboxylic acid(3);
7-[(Z)-2-(2-amino-thiazol-4-yl)-2-methoxyimino-acetylamino]-3-(2,6-d inmino-pyrimidin-4-ylsulfanylmethylsulfanyl)-8-oxo-5-thin-1-aza-bicyc lo[4,2,0]oct-2-ene-2-carboxylic acid(4);
7-[(Z)-2-(2-amino-thiazol-4-yl)-2-hydroxyimino-acetylamino]-3-(4,6-di amino-pyrimidin-2-ylsulfanylmethylsulfanyl)-8-oxo-5-thia-1-aza-bicycl o[4,2,0]oct-2-ene-2-carboxylic acid(5);
7-[(Z)-2-(2-amino-thiazol-4-yl)-2-hydroxyimino-acetylamino]-3-(2,6-di amino-pyrimidin-4-ylsulfanylmethylsulfanyl)-8-oxo-5-thia-1-aza-bicycl o[4,2,0]oct-2-ene-2-carboxylic acid(6);
7-[(Z)-2-(2-amino-5-chloro-thiazol-4-yl)-2-hydroxyimino-acetylamino] -3-(2,6-diamino-pyrimidin-4-ylsulfanylmethylsulfanyl)-8-oxo-5-thia-1-aza-bicyclo[4,2,0]oct-2-ene-2-carboxylic acid(7);
7-[(Z)-2-(2-amino-5-chloro-thiazol-4-yl)-2-methoxyimino-acetylamino] -3-(2,6-diamino-pyrimidin-4-ylsulfanylmethylsulfanyl)-8-oxo-5-thia-1-aza-bicyclo[4,2,0]oct-2-ene-2-carboxylic acid(8);
7-[(Z)-2-(2-amino-thiazol-5-chloro-4-yl)-2-hydroxyimino-acetylamino] -3-(2-amino-6-methyl-pyrimidin-4-ylsulfanylmethylsulfanyl)-8-oxo-5-t hia-1-aza-bicyclo[4,2,0]oct-2-ene-2-carboxylic acid(9);
7-[(Z)-2-(2-amino-thiazol-5-chloro-4-yl)-2-hydroxyimino-acetylamino] -3-(4-amino-pyrimidin-2-ylsulfanylmethylsulfanyl)-8-oxo-5-thia-1-azabicyclo[4,2,0]oct-2-ene-2-carboxylic acid(10);
7-[(Z)-2-(2-amino-thiazol-5-chloro-4-yl)-2-hydroxyimino-acetylamino] -3-(2-amino-6-hydroxy-pyrimidin-4-ylsulfanylmethylsulfanyl)-8-oxo-5-thia-1-aza-bicyclo[4,2,0]oct-2-ene-2-carboxylic acid(11);
7-[(Z)-2-(2-amino-thiazol-5-chloro-4-yl)-2-hydroxyimino-acetylamino] -3-(4,6-diamino-pyrimidin-2-ylsulfanylmethylsulfanyl)-8-oxo-5-thia-1-aza-bicyclo[4,2,0]oct-2-ene-2-carboxylic acid(12);
7-[(Z)-2-(2-amino-thiazol-5-chloro-4-yl)-2-hydroxyimino-acetylamino] -3-(4-amino-6-hydroxy-pyrimidin-2-ylsulfanylmethylsulfanyl)-8-oxo-5-thia-1-aza-bicyclo[4,2,0]act-2-ene-2-carboxylic acid(13);
7-[(Z)-2-(2-amino-thiazol-5-chloro-4-yl)-2-hydroxyimino-acetylamino] -3-(4,5-diamino-2-hydroxy-pyrimidin-6-ylsulfanylmethylsulfanyl)-8-ox o-5-thia-1-aza-bicyclo[4,2,0]oct-2-ene-2-carboxylic acid(14);
7-[(Z)-2-(2-amino-thiazol-5-chloro-4-yl)-2-hydroxyimino-acetylamino] -3-(2,5-diamino-6-hydroxy-pyrimidin-4-ylsulfanylmethylsulfanyl)-8-ox o-5-thia-1-aza-bicyclo[4,2,0]oct-2-ene-2-carboxylic acid(15);
7-{[2-(2-amino-5-chloro-1,3-thiazol-4-yl)-2-(methoxyimino)acetyl]ami no}-3-({[(2,6-diamino-4-pyrimidinyl)sulfanyl]methyl}sulfanyl)-8-oxo-5-thia-1-aza-bicyclo[4,2,0]oct-2-ene-2-carboxylic acid (E)-2-(ethoxycarbonyl)-2-pentenyl ester(16);
7-{ [2-(2-amino-5-chloro-1,3-thiazol-4-yl)-2-(methoxyimino)acetyl]ami no}-3-({[(2,6-diamino-4-pyrimidinyl)sulfanyl]methyl}sulfanyl)-8-oxo-5-thia-1-aza-bicyclo[4,2,0]oct-2-ene-2-carboxylic acid [(2,2-dimethylpropanoyl)oxy]methyl ester(17);
7-{[2-(2-{[(2S)-2-amino-2-phenylacetyl]amino}-5-chloro-1,3-thiazol-4-yl)-2-(methoxyimino)acetyl]amino}-3-({[(2,6-diamino-4-pyrimidinyl)s ulfanyl]methyl}sulfanyl)-8-oxo-5-thia-1-aza-bicyclo[4,2,0]oct-2-ene-2-carboxylic acid(18);
7-[(Z)-2-(2-amino-1,3-thiazol-4-yl)-2-methoxyimino-acetylamino]-3-(2 ,4-diaminopyrimidin-5-ylmethylsulfanyl)-8-oxo-5-thia-1-aza-bicyclo[4, 2,0]oct-2-ene-2-carboxylic acid(19);
7-[(Z)-2-(2-amino-1,3-thiazol-4-yl)-2-methoxyimino-acetylamino]-3-(2 ,4-diaminopyrmudin-6-ylmethylsulfanyl)-8-oxo-5-thia-1-aza-bicyclo[4, 2,0]oct-2-ene-2-carboxylic acid(20);
7-[(Z)-2-(2-amino-5-chloro-1,3-thiazol-4-yl)-2-methoxyimino-acetylam ino]-3-(2,4-diaminopyrimidin-5-ylmethylsulfanyl)-8-oxo-5-thia-1-aza-b icyclo[4,2,0]oct-2-ene-2-carboxylic acid(21);
7-[(Z)-2-(2-amino-5-chloro-1,3-thiazol-4-yl)-2-hydroxyimino-acetylami no]-3-(2,4-diaminopyrimidin-6-ylmethylsulfanyl)-8-oxo-5-thia-1-aza-bi cyclo[4,2,0]oct-2-ene-2-carboxylic acid(22);
7-[(Z)-2-(2-amino-1,3-thiazol-4-yl)-2-methoxyimino-acetylamino]-3-(2 ,4-diaminopyrimidin-5-ylmethylsulfanylmethyl)-8-oxo-5-this-1-aza-bic yclo[4,2,0]oct-2-ene-2-carboxylic acid(23);
7-[(Z)-2-(2-amino-5-chloro-1,3-thiazol-4-yl)-2-hydroxyimino-acetylami no]-3-(2,4-diaminopyrimidin-5-ylmethylsulfanylmethyl)-8-oxo-5-thia-1 -aza-bicyclo[4,2,0]oct-2-ene-2-carboxylic acid(24).

3. A process for preparing cephalosporin compounds of the formula (I) as defined in claim 1, **characterized in that**
(a) compounds of the following formula (V-a): 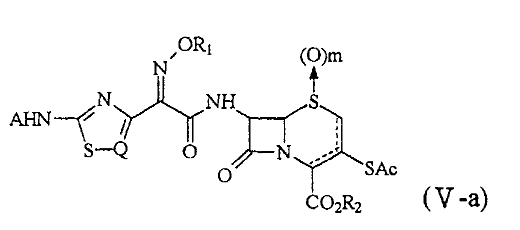 wherein A, R₁, R₂, and Q are each as defined in claim 1 and m is 0 or 1, are reacted in the presence of base with compounds of the following formula (VI-a): 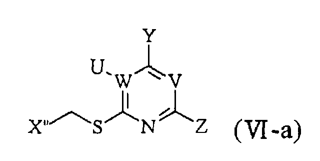 , wherein U, V, W, Y and Z are each as defined in claim 1 and X" is halogen atom, to prepare compounds of the following formula (I-a): 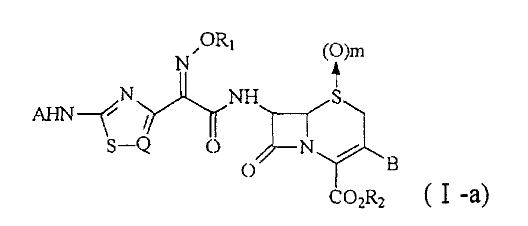 , wherein A, B, R₁, R₂, and Q are each as defined in claim 1 and m is as defined above,
(b) compounds of the following formula (V-b): 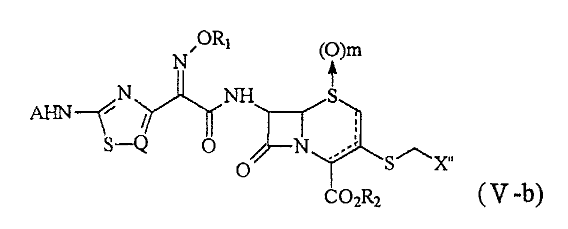 , wherein A, R₁, R₂, and Q are each as defined in claim 1 and X" and m are each as defined above, are reacted with compounds of the following formula (VI-b): 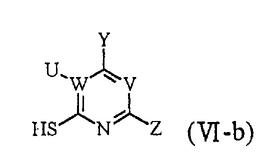 , wherein U, V, W, Y, and Z are each as defined in claim 1, to prepare compounds of the above formula ( I -a),
(c) compounds of the above formula (V-a) are reacted in the presence of base with compounds of the following formula (VI-c): 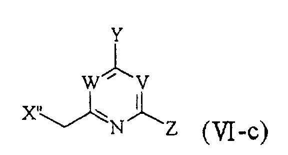 , wherein V, W, Y, Z are each as defined in claim 1 and X" is as defined above, or compounds of the following formula (VI-d): 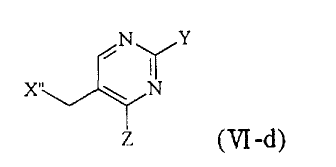 , wherein Y and Z are each as defined in claim 1 and X" is as defined above, to prepare compounds of the above formula (I-a),
(d) compounds of the following formula (V-c): 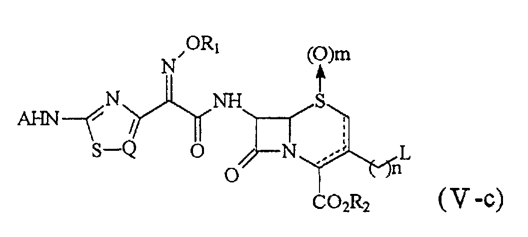 , wherein A, R₁, R₂, Q and n are each as defined in claim 1, m is as defined above and L is leaving group, are reacted with in the presence of base with compounds of the following formula (VI-e): 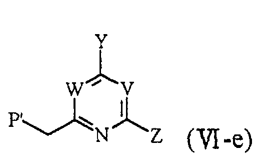 , wherein V, W, Y and Z are each as defined in claim 1 and P' is thiol, alkyl- or arylthioester, or compounds of the following formula (VI-f): 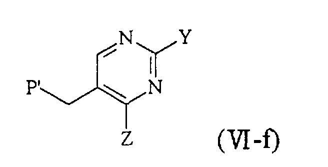 , wherein Y and Z are each as defined in claim 1 and P' is as defined above, to prepare compounds of the above formula ( I -a),
(e) compounds of the above formula (V-c) are reacted with compounds of the above formula (VI-b) to prepare compounds of the above formula ( I -a), or
(f) compounds of the above formula ( I -a), wherein m is 1, are reduced to compounds of the above formula (I).

4. Antimicrobial compositions containing the compounds of the formula (I) as defined in claim 1 as active ingredients and pharmaceutically acceptable carriers.

5. The antimicrobial compositions according to claim 4 which are formulated into injectable or orally administrable dosage forms.

## Patentansprüche

1. Cephalosporinverbindungen mit der folgenden Formel (I): 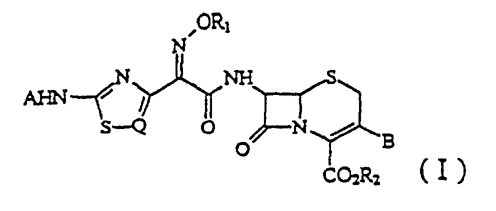 , wobei
A Wasserstoff oder Aminoschutzgruppe repräsentiert,
R₁ Wasserstoff, C₁₋₆alkyl, das durch C₁₋₆alkoxy mono- bis tri- substituiert sein kann, Cyano, Halogen, C₃₋₆Cycloalkyl oder C₅₋₆Heteroaryl mit 1 oder 2 Stickstoff- oder Sauerstoffatom(en), C₃₋₄Alkenyl, C₃₋₄Alkinyl, daß durch Amino substituiert sein kann, oder C₃₋₆Cycloalkyl repräsentiert,
R₂ Wasserstoff oder eine Carboxyschutzgruppe repräsentiert,
Q CH, CX oder N, wobei X Halogen repräsentiert, repräsentiert,
B ein Radikal repräsentiert, ausgewählt aus der folgenden Gruppe: 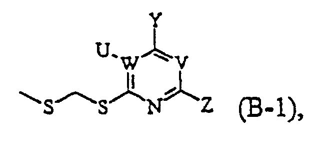 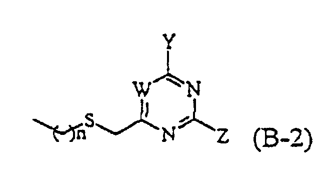 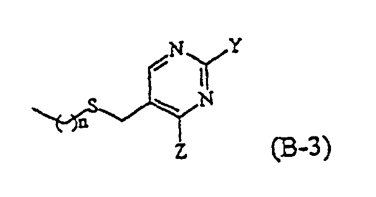 , wobei
U Wasserstoff oder optional substituiertes Amino repräsentiert,
V und W unabhängig voneinander C oder N repräsentieren, mit dem Vorbehalt, daß V und W unterschiedlich sind,
Y und Z unabhängig voneinander Wasserstoff, optional substituiertes Amino, Hydroxy oder C₁₋₄Alkyl repräsentieren,
und
n 0 oder 1 ist,
pharmazeutisch akzeptable nicht-toxische Salze, physiologisch hydrolysierbare Ester, Hydrate, Solvate oder Isomere davon.

2. Die Cephalosporinverbindungen nach Anspruch 1, die ausgewählt sind aus der Gruppe bestehend aus:
7 - [ (Z) - 2 - (2 - Amino - thiazol - 4 - yl) - 2 - methoxyimino - acetylamino] - 8 - oxo - 3 - (pyrimidin - 2 - ylsulfanylmethylsulfanyl) - 5 - thia - 1 - aza - bicyclo [4,2,0] oct 2 - en - 2 - carbonsäure (1);
7 - [(Z) - 2 - (2 - Amino - thiazol - 4 - yl) - 2 - methoxyimino - acetylamino] - 8 - oxo - 3 - (4 - amino - pyrimidin - 2 - ylsulfanylmethylsulfanyl) - 5 - thia - 1 - aza - bicyclo [4,2,0] oct - 2 - en - 2 - carbonsäure (2) ;
7 - [(Z) - 2 - (2 - amino - thiazol - 4 - yl) - 2 - methoxyimino - acetylamino] - 3 - (4,6 - diamino - pyrimidin - 2 - ylsulfanylmethylsulfanyl) - 8 - oxo - 5 - thia - 1 - aza - bicyclo [4,2,0] oct - 2 - en - 2 - carbonsäure (3) ;
7 - [(Z) - 2 - (2 - amino - thiazol - 4 - yl) - 2 - methoxyimino - acetylamino] - 3 - (2,6 - diamino - pyrimidin - 4 - ylsulfanylmethylsulfanyl - 8 - oxo,- 5 - thia - 1 - aza - bicyclo [4,2,0] oct - 2 - en - 2 - carbonsäure (4);
7 - [(Z) - 2 - (2 - amino - thiazol - 4 - yl) - 2 - hydroxyimino - acetylamino] - 3 - (4,6 - diamino - pyrimidin - 2 - ylsulfanylmethylsulfanyl) - 8 - oxo - 5 - thia - 1 - aza - bicyclo [4,2,0] oct - 2 - en - 2 - carbonsäure (5) ;
7 - [(Z) - 2 - (2 - amino - thiazol - 4 - yl) - 2 - hydroxyimino - acetylamino] - 3 - (2,6 - diamino - pyrimidin - 4 - ylsulfanylmethylsulfanyl) - 8 - oxo - 5 - thia - 1 - aza - bicyclo [4,2,0] oct - 2 - en - 2 - carbonsäure (6) ;
7 - [(Z) - 2 - (2 - Amino - 5 - chloro - thiazol - 4 - yl) - 2 - hydroxyimino - acetylamino] - 3 - (2,6 - diamino - pyrimidin - 4 - ylsulfanylmethylsulfanyl) - 8 - oxo - 5 - thia - laza - bicyclo [4,2,0] oct - 2 - en - 2 - carbonsäure (7);
7 - [(Z) - 2 - (2 - Amino - 5 - chloro - thiazol - 4 - yl) - 2 - methoxyimino - acetylamino] - 3 - (2,6 - diamino - pyrimidin - 4 - ylsulfanylmethylsulfanyl) - 8 - oxo - 5 - thia - 1 - aza - bicyclo [4,2,0] oct - 2 - en - 2 - carbonsäure (8);
7 - [(Z) - 2 - (2 - Amino - thiazol - 5 - chloro - 4 - yl) - 2 - hydroxyimino - acetylamino] - 3 - (2 - amino - 6 - methyl - pyrimidin - 4 - ylsulfanylmethylsulfanyl) - 8 - oxo - 5 - thia - 1 - aza - bicyclo [4,2,0] oct - 2 - en - 2 - carbonsäure (9) ;
7 - [(Z) - 2 - (2 - Amino - thiazol - 5 - chloro - 4 - yl) - 2 - hydroxyimino - acetylamino] - 3 - (4 - amino - pyrimidin - 2 - ylsulfanylmethylsulfanyl) - 8 - oxo - 5 - thia - 1 - aza - bicyclo [4,2,0] oct - 2 - en - 2 - carbonsäure (10) ;
7 - [(Z) - 2 - (2 - Amino - thiazol - 5 - chloro - 4 - yl) - 2 - hydroxyimino - acetylamino] - 3 - (2 - amino - 6 - hydroxy - pyrimidin - 4 - ylsulfanylmethylsulfnayl)-8 - oxo - 5 - thia - 1 - aza - bicyclo [4,2,0] oct - 2 - en - 2 - carbonsäure (11) ;
7 - [(Z) - 2 - (2 - Amino - thiazol - 5 - chloro - 4 - yl) - 2 - hydroxyimino - acetylamino] - 3 - (4,6 - diamino - pyrimidin - 2 - ylsulfanylmethylsulfanyl) - 8 - oxo - 5 - thia - 1 - aza - bicyclo [4,2,0] oct - 2 - en - 2 - carbonsäure (12) ;
7 - [(Z) - 2 - (2 - Amino - thiazol - 5 - chloro - 4 - yl) - 2 - hydroxyimino - acetylamino] - 3 - (4 - amio - 6 - hydroxy - pyrimidin - 2 - ylsulfanylmethylsulfanyl) - 8 - oxo - 5 - thia - 1 - aza - bicyclo [4,2,0] oct - 2 - en - 2 - carbonsäure (13);
7 - [(Z) - 2 - (2 - Amino - thiazol - 5 - chloro - 4 - yl) - 2 - hydroxyimino - acetylamino] - 3 - (4,5 - diamino - 2 - hydroxy - pyrimidin - 6 - ylsulfanylmethylsulfanyl) - 8 - oxo - 5 - thia - 1 - aza - bicyclo [4,2,0] oct - 2 - en - 2 - carbonsäure (14);
7 - [(Z) - 2 - (2 - Amino - thiazol - 5 - chloro - 4 - yl) - 2 - hydroxyimino - acetylamino] - 3 - (2,5 - diamino - 6 - hydroxy - pyrimidin - 4 - ylsulfanylmethylsulfanyl) - 8 - oxo - 5 - thia - 1 - aza - bicyclo [4,2,0] oct - 2 - en - 2 - carbonsäure (15);
7 - {[2 - (2 - Amino - 5 - chloro - 1,3 - thiazol - 4 - yl) - 2 - (methoxyimino)acetyl]amino} - 3 - ({[(2,6 - diamino - 4 - pyrimidinyl)sulfanyl]methyl}sulfanyl) - 8 - oxo - 5 - thia - 1 - aza - bicyclo [4,2,0] oct - 2 - en - 2 - carbonsäure (E) - 2 - (ethoxycarbonyl) - 2 - pentenylester (16);
7 - {[2 - (2 - Amino - 5 - chloro - 1,3 - thiazol - 4 - yl) - 2 - (methoxyimino)acetyl]amino} - 3 - ({[(2,6 - diamino - 4 - pyrimidinyl)({[(2,6- diamino - 4 - pyrimidinyl)sulfanyl]methyl}sulfanyl) - 8 - oxo - 5 - thia - 1 - aza - bicyclo [4,2,0] oct - 2 - en - 2 - carbonsäure[(2,2 - dimethylpropanoyl)oxy]methylester (17);
7 - {[2 - (2 - {[(2S) - 2 - Amino - 2 - phenylacetyl]amino} - 5 - chloro - 1,{[2 - (2 - {[(2S) - 2 - amino - 2 - phenylacetyl]amino} - 5 - chloro - 1,3 - thiazol - 4yl) - 2 - (methoxyimino)acetyl]amino} - 3 - ({[(2,6 - diamino - 4 - pyrimidinyl)sulfanyl]methyl}sulfanyl)- 8 - oxo - 5 - thia - 1 - aza - bicyclo [4,2,0] oct - 2 - en - 2 - carbonsäure (18) ;
7 - [(Z) - 2 - (2 - Amino - 1,3 - thiazol - 4 - yl) - 2 - methoxyimino - acetylamino] - 3 - (2,4 - diaminopyrimidin - 5 - ylmethylsulfanyl) - 8 - oxo - 5 - thia - 1 - aza - bicyclo [4,2,0] oct - 2 - en - 2 - carbonsäure (19);
7 - [(Z) - 2 - (2 - Amino - 1,3 - thiazol - 4 - yl) - 2 - methoxyimino - acetylamino] - 3 - (2,4 - diaminopyrimidin - 6 - ylmethylsulfanyl) - 8 - oxo - 5 - thia - 1 - aza - bicyclo [4,2,0] oct - 2 - en - 2 - carbonsäure (20);
7 - [(Z) - 2 - (2 - Amino - 5 - chloro - 1,3 - thiazol - 4 - yl) - 2 - methoxyimino - acetylamino] - 3 - (2,4 - diaminopyrimidin - 5 - ylmethylsulfanyl) - 8 - oxo - 5 - thia - 1 - aza - bicyclo [4,2,0] oct - 2 - en - 2 - carbonsäure (21) ;
7 - [(Z) - 2 - (2 - Amino - 5 - chloro - 1,3 - thiazol - 4 - yl) - 2 - hydroxyimino - acetylamino] - 3 - (2,4 - diaminopyrimidin - 6 - ylmethylsulfanyl) - 8 - oxo - 5 - thia - 1 - aza - bicyclo [4,2,0] oct - 2 - en - 2 - carbonsäure (22) ;
7 - [(Z) - 2 - (2 - Amino - 1,3 - thiazol - 4 - yl) - 2 - methoxyimino - acetylamino] - 3 - (2,4 - diaminopyrimidin - 5 - ylmethylsulfanylmethyl) - 8 - oxo - 5 - thia - 1 - aza - bicyclo [4,2,0] oct - 2 - en - 2 - carbonsäure (23);
7 - [(Z) - 2 - (2 - Amino - 5 - chloro - 1,3 - thiazol - 4 - yl) - 2 - hydroxyimino - acetylamino] - 3 - (2,4 - diaminopyrimidin - 5 - ylmethylsulfanylmethyl) - 8 - oxo - 5 - thia - 1 - aza - bicyclo [4,2,0] oct - 2 - en - 2 - carbonsäure (24) ;

3. Ein Verfahren, um Cephalosporinverbindungen der Formel (I), wie in Anspruch 1 definiert, herzustellen, **dadurch gekennzeichnet, daß**
(a) Verbindungen der folgenden Formel (V-a) : 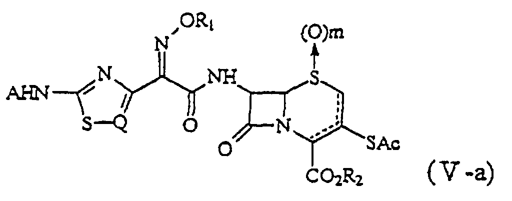 wobei A, R₁, R₂, und Q alle wie in Anspruch 1 definiert sind und m 0 oder 1 ist, werden in der Gegenwart von Base mit Verbindungen der folgenden Formel (VI-a) umgesetzt: 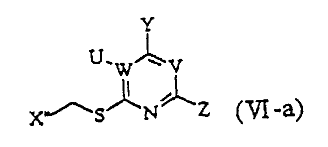 , wobei U, V, W, Y und Z alle wie in Anspruch 1 definiert sind und X" ein Halogenatom ist, um Verbindungen der folgenden Formel herzustellen (I-a): 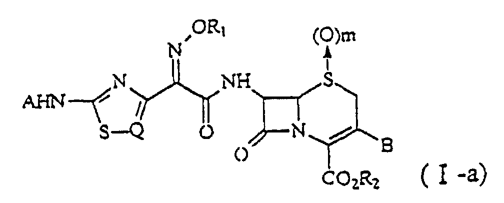 , wobei A, B, R₁, R₂, und Q alle wie in Anspruch 1 definiert sind und m wie oben definiert ist,
(b) Verbindungen der folgenden Formel (V-b) : 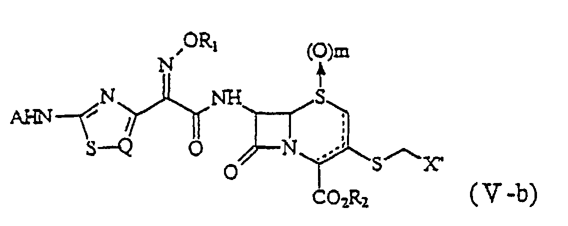 , wobei A, R₁, R₂, und Q alle wie in Anspruch 1 definiert sind und X" und m beide wie oben definiert sind, werden mit Verbindungen der folgenden Formel (VI-b) umgesetzt: 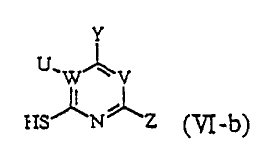 , wobei U, V, W, Y, und Z alle wie in Anspruch 1 definiert sind, um Verbindungen der obigen Formel (I-a) herzustellen,
(c) Verbindungen der obigen Formel (V-a) werden in der Gegenwart von Base mit Verbindungen der folgenden Formel (VI-c) umgesetzt: 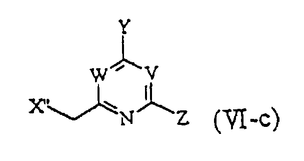 , wobei V, W, Y, Z alle wie in Anspruch 1 definiert sind und X" wie oben definiert ist, oder Verbindungen der folgenden Formel (VI-d): 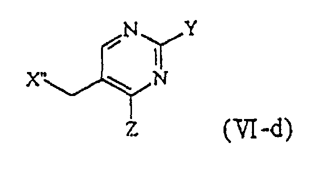 , wobei Y und Z beide wie in Anspruch 1 definiert sind und X" wie oben definiert ist, um Verbindungen der obigen Formel (I-a) herzustellen,
(d) Verbindungen der folgenden Formel (V-c): 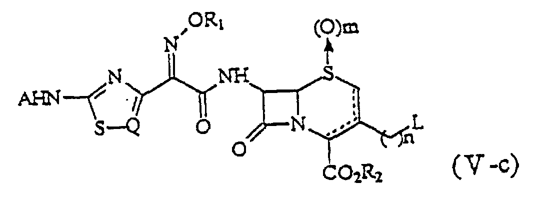 , wobei A, R₁, R₂, Q und n alle wie in Anspruch 1 definiert sind, m wie oben definiert ist und L Abgangsgruppe ist, werden in der Gegenwart von Base mit Verbindungen der folgenden Formel (VI-e) umgesetzt: 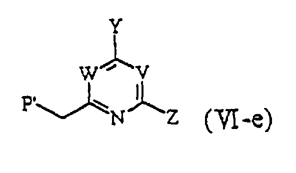 , wobei V, W, Y und Z alle wie in Anspruch 1 definiert sind und P' Thiol, Alkyl-oder Arylthioester ist, oder Verbindungen der folgenden Formel (VI-f) : 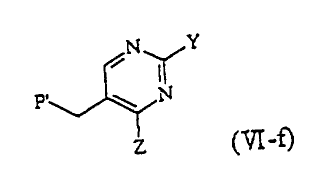 , wobei Y und Z alle wie in Anspruch 1 definiert sind und P' wie oben definiert ist, um Verbindungen der obigen Formel (I-a) herzustellen,
(e) Verbindungen der obigen Formel (V-c) werden mit Verbindungen der obigen Formel (VI-b) umgesetzt, um Verbindungen der obigen Formel (I-a) herzustellen, or
(f) Verbindungen der obigen Formel (I-a), wobei m 1 ist, werden zu Verbindungen der obigen Formel (I) reduziert.

4. Antimikrobielle Zusammensetzungen, die Verbindungen der Formel (I), wie definiert in Anspruch 1 als aktive Bestandteile und als pharmazeutisch akzeptable Trägerstoffe enthalten.

5. Die antimikrobiellen Zusammensetzungen nach Anspruch 4, die in injizierbaren oder oral administrierbaren Dosierformen formuliert sind.

## Revendications

1. Composés de céphalosporine de formule (1) suivante : 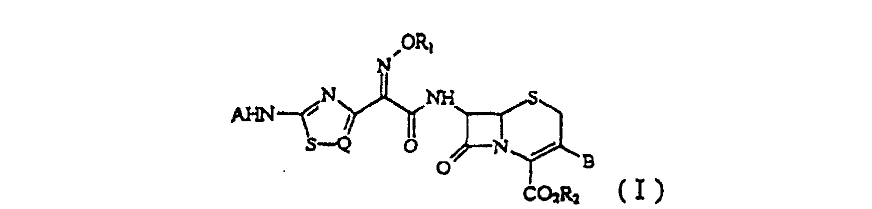 dans laquelle :
- A représente l'hydrogène ou un groupe amino protecteur,
- R₁ représente l'hydrogène, un groupe alkyle C₁₋₆ qui peut être mono- à tri-substitué par les groupes alcoxy C₁₋₆, cyano, halogène, cycloalkyle C₃₋₆ ou hétéroaryle C₅₋₆ ayant 1 ou 2 atome(s) d'azote ou d'oxygène, un groupe alkényle C₃₋₄, alkynyle C₃₋₄ pouvant être substitué par un groupe amino, ou un groupe cycloalkyle C₃₋₆,
- R₂ représente l'hydrogène ou un groupe carboxy protecteur,
- Q représente CH, CX ou N, où X représente un halogène,
- B représente un radical choisi au sein de l'ensemble suivant : 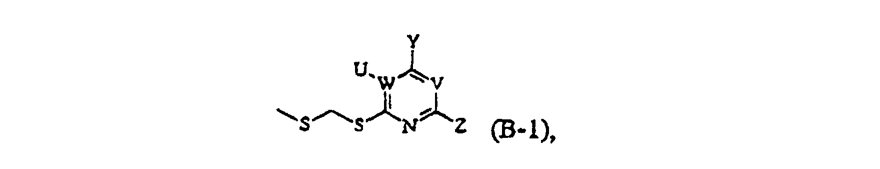 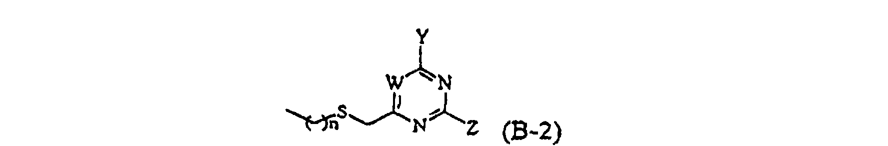 où 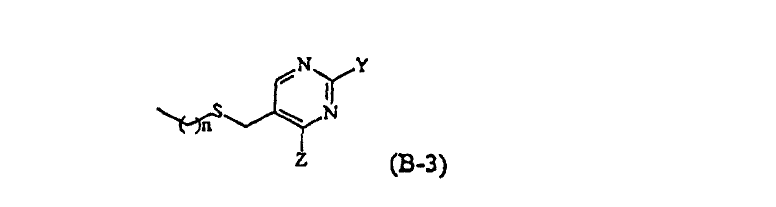
- U représente l'hydrogène ou un groupe amino éventuellement substitué,
- V et W indépendamment l'un de l'autre, représentent C ou N, à la condition que V et W soient différents,
- Y et Z indépendamment l'un de l'autre, représentent l'hydrogène, un groupe amino, hydroxy ou alkyle C₁₋₄ éventuellement substitué, et
- n est égal à 0 ou à 1,
el leurs sels, esters hydrates, solvates ou isomères physiologiquement hydrolysables, non-toxiques, pharmaceutiquement acceptables.

2. Composés de céphalosporine selon la revendication 1, qui sont choisis au sein du groupe comprenant :
- l'acide 7-[(Z)-2-(2-aminothiazol-4-yl)-2-méthoxyiminoacétylamino]-8-oxo-3-(pyrimidin-2-ylsulfanylméthylsulfanyl)-5-thia-1-azabicyclo-[4,2,0]oct-2-ène-2-carboxylique (1);
- l'acide 7-f(Z)-2-(2-aminothiazol-4-yl)-2-méthoxyiminoacétylamino]-8-oxo-3-(4-aminopyrimidin-2-ylsulfanylméthylsulfanyl)-5-thia-1-aza-bicyclo[4,2,0]oct-2-ène-2-carboxylique (2) ;
- l'acide 7-[(Z)-2-(2-aminothiazol-4-yl)-2-méthoxyiminoacétylamino]-3-(4,6-diaminopyrimidin-2-ylsulfanylméthylsulfanyl)-8-oxo-5-thia-1-aza-bicyclo[4,2,0]oct-2-ène-2-carboxylique (3) ;
- l'acide 7-[(Z)-2-(2-aminothiazol-4-yl)-2-méthoxyiminoacétylamino]-3-(2,6-diaminopyrimidin-4-ylsulfanylméthylsulfanyl)-8-oxo-5-thia-1-aza-bicyclo[4,2,0]oct-2-ène-2-carboxylique (4) ;
- l'acide 7-[(Z)-2-(2-aminothiazol-4-yl)-2-hydroxyiminoacétylamino]-3-(4,6-diaminopyrimidin-2-ylsulfanylméthylsulfanyl)-8-oxo-5-thia-1-aza-bicyclo[4,2,0]oct-2-ène-2-carboxylique (5);
- l'acide 7-[(Z)-2-(2-aminothiazol-4-yl)-2-hydroxyiminoacétylamino]-3-(2,6-diaminopyrimidin-4-ylsulfanylméthylsulfanyl)-8-oxo-5-thia-1-aza-bicyclo[4,2,0']oct-2-ène-2-carboxylique (6) ;
- l'acide 7-[(Z)-2-(2-amino-5-chlorothiazol-4-yl)-2-hydroxyiminoacétylamino]-3-(2,6-diaminopyrimidin-4-ylsulfanylméthylsulfanyl)-8-oxo-5-thia-1-aza-bicyclo[4,2,0]oct-2-ène-2-carboxylique (7) ;
- l'acide 7-[(Z)-2-(2-amino-5-chlorothiazol-4-yl)-2-méthoxyiminoacétylamino]-3-(2,6-diaminopyrimidin-4-ylsulfanylméthylsulfanyl)-8-oxo-5-thia-1-azabicyclo[4,2,0]oct-2-ène-2-carboxylique (8) ;
- l'acide 7-[(Z)-2-(2-aminothiazol-5-chloro-4-yl)-2-hydroxyiminoacétylamino]-3-(2-amino-6-méthylpyrimidin-4-ylsulfanylméthylsulfanyl)-8-oxo-5-thia-1-azabicyclo[4,2,0]oct-2-ène-2-carboxylique (9) ;
- l'acide 7-[(Z)-2-(2-aminothiazol-5-chloro-4-yl)-2-hydroxyiminoacétylamino]-3-(4-aminopyrimidin-2-ylsulfanylméthylsulfanyl)-8-oxo-5-thia-1-azabicyclo[4,2,0]oct-2-ène-2-carboxylique (10) ;
- l'acide 7-[(Z)-2-(2-aminothiazol-5-chloro-4-yl)-2-hydroxyiminoacétylamino]-3-(2-amino-6-hydroxypyrimidin-4-ylsulfanylméthylsulfanyl)-8-oxo-5-thia-1-azabicyclo[4,2,0]oct-2-ène-2-carboxylique (11) ;
- l'acide 7-[(Z)-2-(2-aminothiazol-5-chloro-4-yl)-2-hydroxyiminoacétylamino]-3-(4,6-diaminopyrimidin-2-ylsulfanylméthylsulfanyl)-8-oxo-5-thia-1-azabicyclo[4,2,0]oct-2-ène-2-carboxylique (12) ;
- l'acide 7-[(Z)-2-(2-aminothiazol-5-chloro-4-yl)-2-hydroxyiminoacétylamino]-3-(4-amino-6-hydroxypyrimidin-2-ylsulfanylméthylsulfanyl)-8-oxo-5-thia-1-azabicyclo[4,2,0]oct-2-ène-2-carboxylique (13);
- l'acide 7-[(Z)-2-(2-aminothiazol-5-chloro-4-yl)-2-hydroxyiminoacétylamino]-3-(4,5-diamino-2-hydroxypyrimidin-6-ylsulfanylméthylsulfanyl)-8-oxo-5-thia-1-azabicyclo[4,2,0]oct-2-ène-2-carboxylique (14) ;
- l'acide 7-[(Z)-2-(2-aminothiazol-5-chloro-4-yl)-2-hydroxyiminoacétylamino]-3-(2,5-diamino-6-hydroxypyrimidin-4-ylsulfanylméthylsulfanyl)-8-oxo-5-thia-1-azabicyclo[4,2,0]oct-2-ène-2-carboxylique (15) ;
- l'ester (E)-2-(éthoxycarbonyl)-2-pentényle de l'acide 7-{[2-(2-amino-5-chloro-1,3-thiazol-4-yl)-2-(méthoxyimino)acétyl]amino}-3-({[2,6-diamino-4-pyrimidinyl)sulfanyl]méthyl}sulfanyl)-8-oxo-5-thia-1-aza-bicyclo[4,2,0]oct-2-ène-2-carboxylique (16);
- l'ester [(2,2-diméthylpropanoyl)oxy]méthyle de l'acide 7-{[2-(2-amino-5-chloro-1,3-thiazol-4-yl)-2-(méthoxyimino)acétyl]amino}-3-({[2,6-diamino-4-pyrimidinyl)sulfanyl]méthyl}sulfanyl)-8-oxo-5-thia-1-azabicyclo[4,2,0]oct-2-ène-2-carboxylique (17) ;
- l'acide 7-{[2-(2-{[(2S)-2-amino-2-phénylacétyl]amino}-5-chloro-1,3-thiazol-4-yl)-2-(méthoxyimino)acétyl]amino}-3-({[2,6-diamino-4-pyrimidinyl)sulfanyl]méthyl}sulfanyl)-8-oxo-5-thia-1-azabicyclo[4,2,0]oct-2-ène-2-carboxylique (18);
- l'acide 7-[(Z)-2-(2-amino-1,3-thiazol-4-yl)-2-méthoxyiminoacétylamino]-3-(2,4-diaminopyrimidin-5-ylméthylsulfanyl)-8-oxo-5-thia-1-azabicyclo[4,2,0]oct-2-ène-2-carboxylique (19) ;
- l'acide 7-[(Z)-2-(2-amino-1,3-thiazol-4-yl)-2-méthoxyiminoacétylamino]-3-(2,4-diaminopyrimidin-6-ylméthylsulfanyl)-8-oxo-5-thia-1-azabicyclo[4,2,0]oct-2-ène-2-carboxylique (20) ;
- l'acide 7-[(Z)-2-(2-amino-5-chlore-1,3-thiazol-4-yl)-2-méthoxyiminoacétylamino]-3-(2,4-diaminopyrimidin-5-ylméthylsulfanyl)-8-oxo-5-thia-1-azabicyclo[4,2,0]oct-2-ène-2-carboxylique (21) ;
- l'acide 7-[(Z)-2-(2-amino-5-chloro-1,3-thiazol-4-yl)-2-hydroxyiminoacétylamino]-3-(2,4-diaminopyrimidin-6-ylméthylsulfanyl)-8-oxo-5-thia-1-azabicyclo[4,2,0]oct-2-ène-2-carboxylique (22) ;
- l'acide 7-[(Z)-2-(2-amino-1,3-thiazol-4-yl)-2-méthoxyiminoacétylamino]-3-(2,4-diaminopyrimidin-5-ylméthylsulfanylméthyl)-8-oxo-5-thia-1-azabicyclo[4,2,0]oct-2-ène-2-carboxylique (23);
- l'acide 7-[(Z)-2-(2-amino-5-chloro-1,3-thiazol-4-yl)-2-hydroxyiminoacétylamino]-3-(2,4-diaminopyrimidin-5-ylméthylsulfanylméthyl)-8-oxo-5-thia-1-azabicyclo[4,2,0]oct-2-ène-2-carboxylique (24).

3. Procédé pour la préparation de composés de céphalosporine de formule (I) selon la revendication 1, **caractérisé en ce que** :
(a) des composés de formule (V-a) suivante : 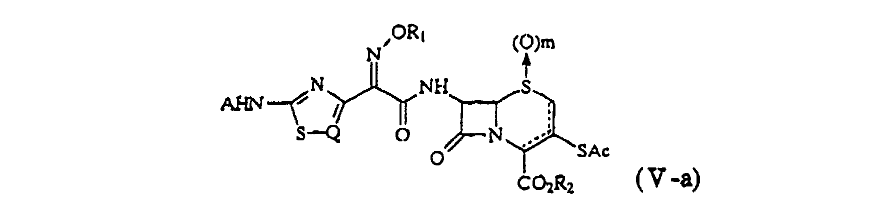 dans laquelle A, R₁, R₂ et Q sont chacun tels que définis dans la revendication 1, et m est égal à 0 ou à 1, sont mis en réaction en présence d'une base avec des composés de formule (VI-a) suivante : 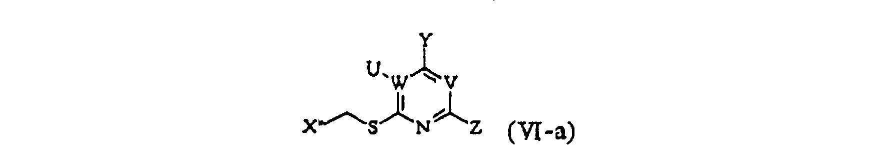 dans laquelle U, V, W, Y et Z sont chacun, tels que définis dans la revendication 1, et X" représente un atome d'halogène, pour préparer des composés de formule (I-a) suivante : 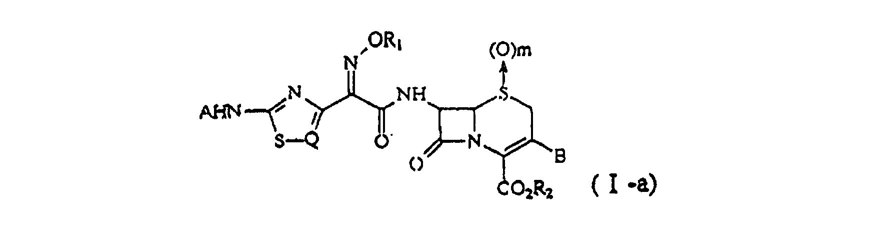 dans laquelle A, B, R₁, R₂ et Q sont chacun, tels que définis dans la revendication 1 et m est tel que défini plus haut,
(b) des composés de formule (V-b) suivante : 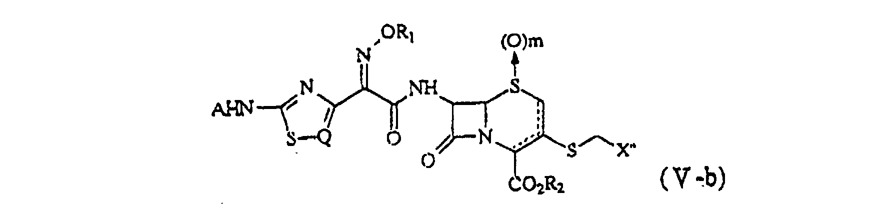 dans laquelle A, R₁, R² et Q sont chacun, tels que définis dans la revendication 1, et X" et m sont chacun tels que définis plus haut, sont mis en réaction avec des composés de formule (V1-b) suivante : 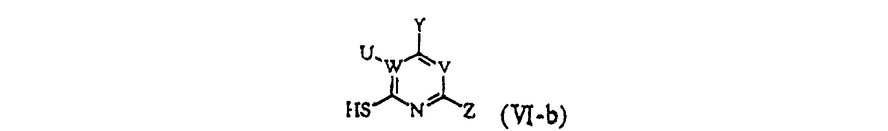 dans laquelle U, V, W, Y et Z sont chacun, tels que définis dans la revendication 1, pour préparer des composés de formule (I-a) ci-dessus,
(c) des composés de formule (V-a) ci-dessus sont mis en réaction en présence d'une base, avec des composés de formule (VI-c) suivante : 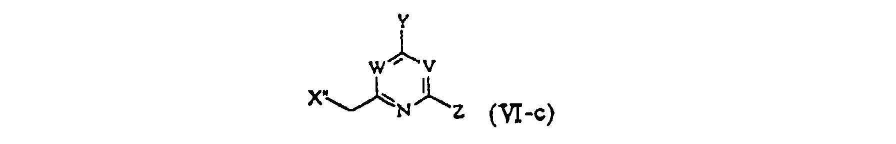 dans laquelle V, W, Y et Z sont chacun, tels que définis dans la revendication 1, et X" est tel que défini plus haut, ou des composés de formule (VI-d) suivante : 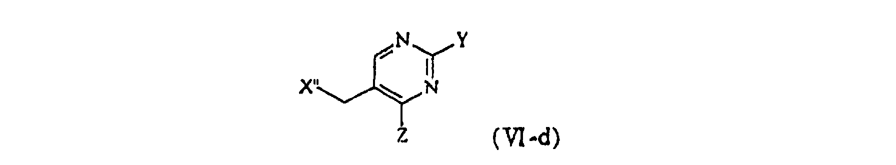 dans laquelle Y et Z sont chacun, tels que définis dans la revendication 1, et X" est tel que défini plus haut,
pour préparer des composés de formule (I-a) ci-dessus,
(d) des composés de formule (V-c) suivante : 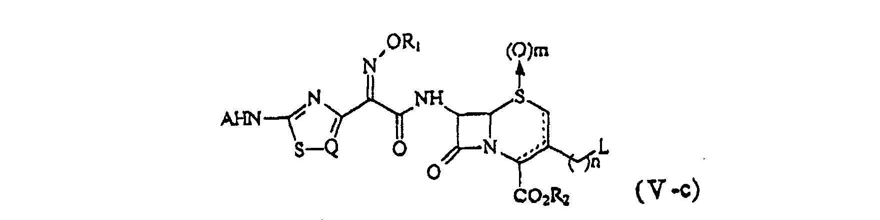 dans laquelle A, R₁, R₂, Q et n sont chacun, tels que définis dans la revendication 1, m est tel que défini ci-dessus, et L représente un groupe partant, sont mis en réaction en présence d'une base, avec des composés de formule (VI-e) suivante : 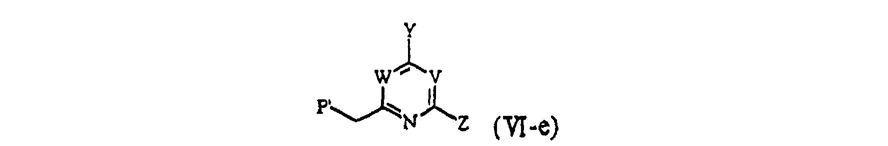 dans laquelle V, W, Y et Z sont chacun, tels que définis dans la revendication I, et P' représente un groupe thiol, alkyl- ou aryl-thioester, ou des composés de formule (VI-f) suivante : 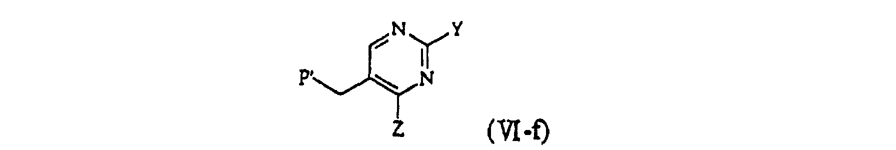 dans laquelle Y et Z sont chacun, tels que définis dans la revendication 1, et P' est tel que défini ci-dessus, pour préparer des composés de formule (I-a) ci-dessus,
(e) des composés de formule (V-c) ci-dessus sont mis en réaction avec des composés de formule (VI-b) ci-dessus, pour préparer des composés de formule (I-a) ci-dessus, ou
(f) des composés de formule (I-a) ci-dessus, dans laquelle m est égal à 1, sont réduits en composés de formule (I) ci-dessus.

4. Compositions antimicrobiennes contenant les composés de formule (I) selon la revendication 1, en tant qu'ingrédients actifs, et des véhicules pharmaceutiquement acceptables.

5. Compositions antimicrobiennes selon la revendication 4, qui sont formulées en formes pharmaceutiques injectables ou administrables par voie orale.
